(19) Europäisches Patentamt / European Patent Office / Office européen des brevets

(11) **EP 4 422 599 B1**

(12) **EUROPEAN PATENT SPECIFICATION**

(45) Date of publication and mention
of the grant of the patent:
**28.01.2026   Bulletin 2026/05**

(21) Application number: **22809383.7**

(22) Date of filing: **24.10.2022**

(51) International Patent Classification (IPC):
*A61K 9/14* (2006.01)      *A61K 9/10* (2006.01)
*A61K 47/36* (2006.01)      *A61K 47/40* (2006.01)
*A61K 31/18* (2006.01)      *A61K 31/4985* (2006.01)
*A61K 45/06* (2006.01)      *A61K 47/38* (2006.01)
*A61K 31/58* (2006.01)      *A61P 13/08* (2006.01)
*A61P 15/10* (2006.01)

(52) Cooperative Patent Classification (CPC):
(C-Sets available)
**A61K 9/0095; A61K 9/10; A61K 9/14; A61K 31/18;
A61K 31/4985; A61K 31/58; A61K 47/36;
A61K 47/38; A61K 47/40; A61P 13/08; A61P 15/10**
(Cont.)

(86) International application number:
**PCT/EP2022/079659**

(87) International publication number:
**WO 2023/072872 (04.05.2023 Gazette 2023/18)**

(54) **TADALAFIL ORAL SUSPENSION**

ORALE SUSPENSION VON TADALAFIL

SUSPENSION ORALE DE TADALAFIL

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2021   EP 21382964**

(43) Date of publication of application:
**04.09.2024   Bulletin 2024/36**

(73) Proprietor: **Farmalider, S.A.**
**28108 Alcobendas (Madrid) (ES)**

(72) Inventors:
• **SANZ MENÉNDEZ, Nuria**
  **E-28108 Alcobendas, Madrid (ES)**
• **MUÑOZ RUIZ, Ángel**
  **E-41012 Sevilla (ES)**

• **SANTÉ SERNA, Luis Narciso**
  **E-28040 Madrid (ES)**
• **PORTOLÉS PÉREZ, Antonio**
  **E-28040 Madrid (ES)**
• **VARGAS CASTRILLÓN, Emilio**
  **E-28040 Madrid (ES)**
• **RUBIO MENCO, Guillermo**
  **E-28108 Alcobendas, Madrid (ES)**
• **HORCAJADA CÓRDOBA, Raquel**
  **E-28108 Alcobendas, Madrid (ES)**
• **DUART GONZÁLEZ, Ester**
  **E-28108 Alcobendas, Madrid (ES)**
• **IGLESIAS SÁNCHEZ, José Carlos**
  **E-28108 Alcobendas, Madrid (ES)**
• **GÓMEZ CALVO, Antonia**
  **E-28108 Alcobendas, Madrid (ES)**

(74) Representative: **ABG Intellectual Property Law,
S.L.**
**Avenida de Burgos, 16D**
**Edificio Euromor**
**28036 Madrid (ES)**

(56) References cited:
EP-A1- 3 498 264     WO-A2-2020/039263

• ALLEN L.V. ET AL.: "Tadalafil 5 mg/mL Oral Suspension", 22 May 2012 (2012-05-22), pages 1 - 5, XP055905249, Retrieved from the Internet <URL:https://www.uspharmacist.com/article/tadalafil-5-mgml-oral-suspension> [retrieved on 20220325]

• BADR-ELDIN S.M. ET AL.: "Inclusion complexes of tadalafil with natural and chemically modified beta-cyclodextrins. I: Preparation and in-vitro evaluation", EUROPEAN JOURNAL OF PHARMACEUTICS AND BIOPHARMACEUTICS, vol. 70, no. 3, 1 November 2008 (2008-11-01), pages 819 - 827, XP055005612, ISSN: 0939-6411, DOI: 10.1016/j.ejpb.2008.06.024

• GUL A. ET AL.: "Effect of tadalafil on penile nitric oxide synthase and corporal smooth muscle in rats under dutasteride treatment", AGING MALE, vol. 23, no. 2, 11 March 2020 (2020-03-11), GB, pages 161 - 167, XP055905429, ISSN: 1368-5538, DOI: 10.1080/13685538.2020.1739019

• SEBASTIANELLI A. ET AL.: "Tadalafil 5 mg Alone or in Combination with Tamsulosin 0.4 mg for the Management of Men with Lower Urinary Tract Symptoms and Erectile Dysfunction: Results of a Prospective Observational Trial", JOURNAL OF CLINICAL MEDICINE, vol. 8, no. 8, 29 July 2019 (2019-07-29), pages 1126, XP055905425, DOI: 10.3390/jcm8081126

(52) Cooperative Patent Classification (CPC): (Cont.)

C-Sets
A61K 31/18, A61K 2300/00;
A61K 31/4985, A61K 2300/00;
A61K 31/58, A61K 2300/00

## Description

### Technical field

The present invention relates to a tadalafil pharmaceutical composition in the form of oral suspension, which is stable and provides equivalent dissolution profile compared to solid oral tadalafil formulations available in the market.

### State of the art

Tadalafil is a selective inhibitor of phosphodiesterase type 5 (PDE5). Inhibition of PDE5 by tadalafil is known to produce increased levels of cyclic guanosine monophosphate (cGMP) in different tissues, in particular, in penile corpus cavernosum under sexual stimulation, and also in prostate, bladder and pulmonary tissues, resulting in relaxation of vascular smooth muscle, increased blood perfusion and/or vasodilation in those tissues. Based on this pharmacological mechanism, tadalafil is used in therapy for the treatment of different conditions, in particular, for erectile dysfunction, for benign prostatic hyperplasia (BPH) and also for pulmonary arterial hypertension (PAH).

The medicament Cialis®, for example, contains tadalafil as active ingredient and is available as film-coated tablets of 2.5, 5, 10 and 20 mg strength. All of them are indicated for the treatment of erectile dysfunction, and the different doses available are intended to fulfil specific patient needs. Usually, a unique dose of either 10 mg or 20 mg taken prior to anticipated sexual activity is recommended. In some cases, however, once daily regimen with lower doses, of 2.5 mg or 5 mg, may be advantageous.

On the other hand, the 5 mg strength tablets are also indicated for the treatment of signs and symptoms of benign prostatic hyperplasia (BPH).

Solid pharmaceutical formulations, as tablets or capsules, are the most common forms available for the administration of active ingredients. However, their use is, under certain circumstances, not completely satisfactory, for example, for patients with swallowing difficulties. For those patients, the availability of alternative liquid formulations may be useful. Furthermore, liquid formulations may provide additional advantages, for example, a more convenient administration, without the need of liquids for swallowing, and the possibility of better tailoring the required dose of the active ingredient, namely, of tadalafil.

However, the preparation of liquid formulations is, in general, more complex than the preparation of standard tablets and capsules and is particularly challenging for those active ingredients, such as tadalafil, which are practically insoluble in water. For those cases, a suspension formulation is, in general, a good choice. However, the formulation of chemically and physically stable suspensions, i.e., where both chemical degradation and sedimentation issues are minimized, is not trivial. Furthermore, it is also desirable to develop simple formulations with only small amounts of excipients, other than water, both for economy reasons and also to reduce possible risks of adverse effects or intolerance issues related to excipients.

Another challenge in the development of new tadalafil suspension formulations is that it is required that they provide equivalent pharmacokinetic profile as the commercial solid formulations which are already available in the market, namely as Cialis® film-coated tablets. For low solubility drugs, as tadalafil, dissolution is typically the absorption limiting step and, therefore, *in vitro* dissolution studies generally provide good correlation with the *in vivo* absorption behaviour. In this regard, therefore, a tadalafil suspension formulation providing *in vitro* dissolution profile similar to that of Cialis® film-coated tablets would be a primary target.

On the other hand, for treating certain conditions, the combined use of tadalafil with other active ingredients may be advantageous. In particular, for example, the combination of tadalafil and tamsulosin, which is an adrenergic $\alpha$1-receptor antagonist, has been widely reported in the art for treating patients with lower urinary tract symptoms (LUTS) related to benign prostatic hyperplasia (BPH), for example, as disclosed in Sebastianelli et al., J. Clin. Med., 2019, 8, 1126. Additionally, the advantages of combining tadalafil and dutasteride, which is a testosterone 5$\alpha$-reductase inhibitor, have also been disclosed in the art, for example, as disclosed in Özkιdιk *et al.,* Turk. J. Urol., 2018, 44, 294.

It would be therefore desirable for a tadalafil suspension formulation to encompass also certain useful fixed dose combinations with other drugs, in particular, with tamsulosin or with dutasteride. These kinds of combined formulations, however, would be particularly troublesome to prepare due to the different physicochemical characteristics and administration requirements of each of the combined drugs. In particular, for example, the combination tadalafil-tamsulosin is particularly challenging as tamsulosin is known to be preferably provided in delayed-release form, as it provides a more consistent release of tamsulosin (Prabhu et al., Drugs Ther. Perspect., 2019, 35, 181-184).

Some tadalafil suspensions have been disclosed in the prior art. For example, the international patent application WO-A-2020/039263 relates to oral suspension formulations of insoluble drugs, in general, including, for example, quetiapine, sildenafil, tadalafil, cinacalcet, ticagrelor, mycophenolate, aprepitant, zonisamide or pirimidone. Wide lists of suitable excipients are provided, including suspending agents, binders/fillers and buffering agents. For drugs having poor wettability and log P higher than 2.5, such as tadalafil, the suspensions contain 300-400 mg/ml of glycerin.

The international patent application WO-A-2018/142189 discloses a method of manufacturing stable suspensions of nanoparticles or tadalafil or sildenafil citrate, of improved bioavailability. The method comprises preparing a solution of the active ingredient in dimethyl sulfoxide (DMSO) and adding this solution, under continuous homogenization, to an aqueous solution of a dispersing agent, which is, preferably, lecithin, gelatin, starch or their combinations.

The proposals available so far in the art fail to fulfil all the desired requirements for a tadalafil suspension. Therefore, there is still the need of providing tadalafil suspensions, which are chemically and physically stable, which are simple, and which provide an *in vitro* dissolution profile equivalent to Cialis® commercial film-coated tablets.

## Object of the invention

The object of the invention is a tadalafil pharmaceutical composition in the form of an aqueous oral suspension.

Another aspect of the invention is a fixed-dose combination dosage form comprising said tadalafil pharmaceutical composition and tamsulosin.

Another aspect of the invention is a fixed-dose combination dosage form comprising said tadalafil pharmaceutical composition and dutasteride.

Another aspect of the invention is a process for the preparation of said tadalafil pharmaceutical composition.

Another aspect of the invention is the tadalafil composition and the fixed-dose combination dosage forms for use in medicine.

## Detailed description of the invention

The object of the present invention is a pharmaceutical composition for oral administration in the form of aqueous suspension which comprises:

a) tadalafil in an amount comprised between 0.01 % w/v and 5 % w/v;
b) cyclodextrin;
c) coprocessed microcrystalline cellulose and sodium carboxymethyl cellulose (MCC-NaCMC);
d) xanthan gum; and
e) water.

The authors of the present invention have developed a liquid pharmaceutical composition in the form of aqueous suspension which comprises tadalafil as active ingredient, and which comprises the combination of cyclodextrin, coprocessed MCC-NaCMC and xanthan gum, which, surprisingly, not only has optimal physicochemical characteristics, but also provides an *in vitro* dissolution profile which is very similar to that of commercially available Cialis® film-coated tablets.

Along the present description, as well as in the claims, the singular expressions, generally preceded by the articles "a", "an" or "the", are meant to include also the plural forms, unless the context clearly indicates otherwise. Furthermore, numeric values preceded by the term "about" or "approximately" are meant to include the exact stated value and also a certain variation around such value, namely a variation or $\pm5\%$ of the stated amount. Numeric ranges defined by lower and upper endpoints are meant to include also said stated endpoints.

Unless otherwise stated, along the present description, as well as in the claims, the percentages disclosed for each component of the composition are in weight/volume (% w/v), i.e., grams of each component in 100 ml of the composition.

The excipients used for preparing the composition of the present invention are well known in the art, and widely available, and are described, for example, in the reference book P.J. Sheskey, W.G. Cook and C.G. Cable, Handbook of Pharmaceutical Excipients, Eighth Edition, Pharmaceutical Press, 2017 [ISBN: 978-0-8571-1271-2]. Also, common excipients and procedures for preparing the compositions are described in the book J.P Remington and A. R. Genaro, Remington, The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 4th edition, Churchill Livingstone Elsevier, 2013 [ISBN: 978-0-7020-4290-4].

### Tadalafil

The pharmaceutical composition of the present invention comprises tadalafil as the active ingredient.

Tadalafil is the International Non-proprietary Name (INN) for the product (6*R*,12a*R*)-6-(benzo[d][1,3]dioxol-5-yl)-2-methyl-2,3,12,12a-tetrahydropyrazino[1',2':1,6] pyrido[3,4-b]indole-1,4(6*H*,7*H*)-dione (CAS 171596-29-5).

Tadalafil may be used in the composition as such, i.e., as the free base, or in the form a pharmaceutically acceptable salt thereof. In a preferred embodiment, tadalafil is used as the free base.

Methods for preparing tadalafil are known in de art, for example, as disclosed in Chapter 38 (Drugs for Treatment of Erectile disfunction) of the book R. Vardanyan and V. Hruby, Synthesis of best-seller drugs, Elsevier, 2016. Tadalafil is also commercially available from several suppliers.

The composition of the invention comprises tadalafil in the form of an aqueous suspension, at a concentration comprised between 0.01% and 5.0%, preferably comprised between 0.02% and 2.0%, more preferably comprised between 0.05% and 1.5%, still more preferably comprised between 0.08% and 1.2%, still more preferably comprised between 0.09% and 1.1% and still more preferably comprised between 0.1% and 1.0%.

The authors of the present invention have found that the suspensions of the present invention are chemically and physically stable along said range of tadalafil concentrations, and this fact allows the preparation of suspensions of different concentrations of the active ingredient, according to the specific therapeutic needs.

In one particular embodiment, the concentration of tadalafil is comprised between 0.01% and 0.40%, preferably comprised between 0.02% and 0.30%, more preferably comprised between 0.05% and 0.20%, still more preferably comprised between 0.08% and 0.15%, still more preferably comprised between 0.09% and 0.12% and still more preferably is about 0.1%.

In another particular embodiment, the concentration of tadalafil is comprised between 0.1% and 4.0%, preferably comprised between 0.2% and 3.0%, more preferably comprised between 0.5% and 2.0%, still more preferably comprised between 0.8% and 1.5%, still more preferably comprised between 0.9% and 1.2%, and still more preferably is about 1.0%.

The size of the tadalafil particles to be used in the compositions of the present invention is not limiting, and any particle size may be suitable. Said particle size may be expressed, for example, as volume diameter Dv which, as is well known in the art, represents the diameter of a sphere having the same volume as the particle. In particular, for example, the particle size may be expressed as the volume median diameter (Dv90 or Dv0.9) which indicates the diameter where 90% of the product has a smaller particle size. The particle size may be determined as volume diameter according to methods known to the skilled person such as laser diffraction based on the use of suitable apparatus such as, for example, Malvern Mastersizer 3000 (Malvern Instruments Ltd., Malvern, Worcestershire, UK), among others.

Tadalafil particles suitable to be used in the present composition may have a Dv90 value comprised between 10 and 100 microns, for example. In particular embodiments, the tadalafil particles have Dv90 < 50 microns, or Dv90 < 40 microns, or Dv90 < 20 microns, or Dv90 < 15 microns.

Cyclodextrin

Cyclodextrins, as is well-known in the art, are cyclic oligosaccharides derived from starch containing at least six D-(+)-glucopyranose units attached by (1→4) glucoside bonds. Among the most commonly used forms are $\alpha$-, $\beta$-, and $\gamma$-cyclodextrin, which have respectively 6, 7, and 8 glucose units, respectively. Highly-branched cyclodextrins (HBCD) having large molecular weight, typically of about 462 kDa, are also available and are also included within the cyclodextrin class.

The term cyclodextrin is meant to include also cyclodextrin derivatives, typically, methyl cyclodextrins, 2-hydroxyethyl cyclodextrins or 2-hydroxypropyl cyclodextrins, among others. Thus, for example, dimethyl-$\beta$-cyclodextrin, trimethyl-$\beta$-cyclodextrin, 2-hydroxyethyl-$\beta$-cyclodextrin, 2-hydroxypropyl-$\beta$-cyclodextrin and sulfobutylether-$\beta$-cyclodextrin are meant to be included within the $\beta$-cyclodextrin group.

Cyclodextrins are typically manufactured by the enzymatic degradation of starch using specialized bacteria, and are widely available from commercial suppliers.

HBCD, in particular, is produced from waxy corn-starch by a cyclisation reaction of a branching enzyme, for example, as disclosed in Tanaka et al., Carbohydr. Res., 1996, 295, 91-101, or in Takata et al., J. Bacteriol., 1996, 178, 1600-1606, or in Takata et al., J. Ferment. Bioeng., 1997, 84, 119-123, or in Fuji et al., Biocatal. Biotransformation, 2003, 21, 167-172. HBCD is also commercially available from different suppliers, for example, with the name Cluster Dextrin®, from the company Glico Nutrition.

The amount of cyclodextrin in the composition is typically comprised between 0.01% and 0.25%, preferably comprised between 0.02% and 0.20%, and more preferably comprised between 0.03% and 0.10%. Preferred concentrations of cyclodextrin are, for example, about 0.03%, or about 0.04%, or about 0.05%, or about 0.06%, or about 0.07%, or about 0.08%, or about 0.09%, or about 0.10%.

Any type of cyclodextrin may be used for preparing the composition of the present invention. In particular, the cyclodextrin may be selected from $\alpha$-cyclodextrin, $\beta$-cyclodextrin, $\gamma$-cyclodextrin, highly-branched cyclodextrin (HBCD), and mixtures thereof. In one embodiment, the cyclodextrin is selected from a $\alpha$-cyclodextrin and a $\beta$-cyclodextrin.

In one embodiment, the cyclodextrin is a highly-branched cyclodextrin (HBCD).

In one embodiment, the cyclodextrin is a $\beta$-cyclodextrin.

In one embodiment, the cyclodextrin is an $\alpha$-cyclodextrin.

In one embodiment, the cyclodextrin is a $\gamma$-cyclodextrin.

Coprocessed microcrystalline cellulose and sodium carboxymethylcellulose

Another component of the pharmaceutical composition according to the present invention is coprocessed microcrystalline cellulose (CMC) and sodium carboxymethylcellulose (NaCMC), also known as microcrystalline cellulose and carmellose sodium (European Pharmacopoeia 7.0), or as microcrystalline cellulose and carboxymethylcellulose sodium (U.S. Pharmacopoeia). It can be abbreviated herein as coprocessed MCC-NaCMC.

MCC-NaCMC as is well known in the art, is a spray- or bulk-dried blend of microcrystalline cellulose and sodium carmellose. It is available commercially, for example, from the company JRS Pharma under the tradename Vivapur® MCG, in several grades, which typically differ in the proportion of MCC and NaCMC and/or in the particle size. The amount of NaCMC in the co-processed MCC-NaCMC is typically not higher than about 22 wt%, typically comprised between about 5 wt% and about 22 wt%. All types and grades of coprocessed MCC-NaCMC are suitable to be used in the present composition. For example, the following grades may be used: Vivapur® MCG 581 P, Vivapur® MCG 591 P, Vivapur® MCG 611 P or Vivapur® MCG 811 P.

The amount of coprocessed CMC-NaCMC in the composition is typically comprised between 0.5% and 2.0%, preferably comprised between 0.6% and 1.4%, more preferably comprised between 0.7% and 1.3%, still more preferably comprised between 0.8% and 1.2%, still more preferably comprised between 0.9% and 1.1%, and still more preferably is about 1.0%.

Xanthan gum

Xanthan gum, as is well-known, is a high molecular weight polysaccharide which is formed by D-glucose, D-mannose and D-glucuronic acid units. It is typically produced by bacteria from the genus *Xanthomonas.*

Xanthan gum can be obtained commercially from many companies, for example, under the name Keltrol® (CP Kelco) or Rhodopol® (Solvay), among many others. It is also available in different grades, according to different particle sizes, and all of them are suitable for the preparation of the suspension according to the present invention.

The amount of xanthan gum in the composition is typically comprised between 0.10% and 1.50%, preferably comprised between 0.20% and 1.00%, more preferably comprised between 0.25% and 0.90%, still more preferably comprised between 0.30% and 0.85%, still more preferably comprised between 0.35% and 0.75%, still more preferably comprised between 0.40% and 0.65%, and still more preferably comprised between 0.45% and 0.60%.

In one embodiment, the amount of xanthan gum is comprised between 0.10% and 0.75%, preferably comprised between 0.20% and 0.60% still more preferably comprised between 0.25% and 0.40%, and still more preferably is about 0.30%.

In one embodiment, the amount of xanthan gum is comprised between 0.10% and 0.75%, preferably comprised between 0.25% and 0.65%, more preferably comprised between 0.36% and 0.54%, still more preferably comprised between 0.40% and 0.50%, and still more preferably is about 0.45%.

In one embodiment, the amount of xanthan gum is comprised between 0.20% and 1.50%, preferably comprised between 0.30% and 0.95%, more preferably comprised between 0.42% and 0.80%, still more preferably comprised between 0.48% and 0.72%, still more preferably comprised between 0.55% and 0.65%, and still more preferably is about 0.60%.

Additional optional ingredients

*Surfactants*

The composition of the present invention may optionally contain a surfactant. Surfactants, as is well known, are amphiphilic molecules, i.e., part of the molecule is hydrophilic and part is lipophilic, and they may be ionic or non-ionic.

Preferably, the composition comprises a surfactant.

In one embodiment, the composition comprises a non-ionic surfactant. It may be selected, for example, from sorbitan fatty acid esters, polyoxyethylene sorbitan fatty acid esters, polyoxyethylene 15 hydroxystearate, polyoxyethylene castor oil derivatives, polyoxyethylene stearates, polyoxyethylene alkyl ethers or polyoxyethylene nonylphenol ethers, among others.

In one embodiment, the composition comprises an anionic surfactant, typically selected from sodium lauryl sulfate and docusate sodium.

When a surfactant is added to the composition according to the invention, it is present in a proportion, which may typically range from about 0.001% to about 2%.

In one preferred embodiment, the composition comprises sodium lauryl sulfate, preferably in an amount comprised between 0.010% and 0.100%, preferably comprised between 0.020% and 0.085%, and more preferably comprised between 0.030% and 0.090%.

*Preservative*

The composition may additionally comprise a preservative to ensure its protection against microbial contamination. Suitable preservatives are, for example, butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate or thimerosal, for example, among others, or mixtures thereof.

Preferably, the composition comprises a preservative.

The amount of preservative generally depends on the particular preservative used, as is well known in the art, and typically may range from about 0.001% to about 5%. The skilled in the art should have no difficulties in selecting the suitable amount in each case, as disclosed in reference books in the art.

In one embodiment the preservative is selected from parabens, benzoic acid and sodium or potassium salts thereof and mixtures thereof, in a concentration typically comprised between about 0.01% and about 1.00%, preferably comprised between 0.05% and 0.50%. In one embodiment, the preservative is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate, and mixtures thereof. In one embodiment the preservative is a paraben or a mixture of parabens, for example, is selected from sodium methyl paraben, sodium propyl paraben and mixtures thereof. In one particular embodiment, the preservative is a mixture of sodium methyl paraben and sodium propyl paraben. In another embodiment, the preservative is selected from sorbic acid, potassium sorbate and mixtures thereof. In another embodiment, the preservative is a mixture of benzoic acid and sodium benzoate.

*pH adjusting agent*

The composition of the present invention may optionally contain a pH adjusting agent to maintain the pH of the suspension in the desired pH range. The pH of the composition is generally comprised between 2.5 and 7, preferably comprised between 4.0 and 6.5, more preferably comprised between 5.5 and 6.

The amount and type of the pH adjusting agent in the composition is such that is suitable to provide the desired pH value, as can be easily determined and adjusted by the skilled in the art.

Preferably, the composition comprises a pH adjusting agent.

If present, the pH-adjusting agent is typically present in an amount comprised in the range of from about 1% to about 4%, preferably from about 1.5% to about 3.5%, and more preferably from about 2% to about 3%.

The pH adjusting agent may be an acidifying agent, such as citric acid, acetic acid, hydrochloric acid, lactic acid, phosphoric acid, or sulfuric acid, for example, and/or an alkalinizing agent, such as ammonia solution, diethanolamine, monoethanolamine, potassium bicarbonate, sodium bicarbonate, potassium citrate, sodium citrate, sodium bicarbonate, or sodium borate, for example. Generally, said acidifying and alkalinizing agents are used in combination, as buffering agent, generally comprising an acid and its conjugated base, for example, citrate buffer, acetate buffer, citrate-phosphate buffer, Tris buffer, or phosphate buffer, among others.

A preferred buffering agent is citrate buffer, which typically may be prepared with sodium citrate and citric acid, for example using sodium citrate and anhydrous citric acid in a weight ratio from 8:1 to 5:1, preferably of about 7:1.

*Sweetening agents*

Optionally, the composition may comprise a sweetening agent to improve the palatability of the suspension, for example, an intense sweetening agent may be used. Intense sweetening agents are generally meant those non-nutritive, high-intensity sweeteners, which are generally from about 100 to about 13000 times sweeter than sucrose.

Suitable intense sweetening agents for use in the present composition are, for example, aspartame, acesulfame potassium, alitame, neohesperidin dihydrochalcone, neotame, saccharin, saccharin sodium, sucralose, thaumatin, or mixtures thereof. Preferably, the sweetening agent is selected from aspartame, acesulfame potassium, saccharin and saccharin sodium.

The amount of the sweetening agent may widely vary depending on the sweetening intensity of the agent, but generally is comprised in the range 0.0001% to 0.5%.

*Flavouring agents*

The composition may optionally additionally comprise a flavouring agent for providing a pleasant flavour and/or odour to the composition. Suitable flavouring agents include natural and artificial flavours. Natural flavours include natural oils, and extracts from plants, leaves, flowers and fruits. Some suitable flavours are, for example, menthol, cinnamon, clove, anise, eucalyptus, peppermint, spearmint, thyme, vanilla, chocolate, fruit flavours, such as cherry flavour, grape flavour, orange flavour, banana flavour, strawberry flavour, lemon flavour, apple flavour, peach flavour, raspberry flavour, pineapple flavour and apricot flavour among many others, and combinations thereof.

The amount of flavouring agent can be easily adjusted by the skilled formulator, depending on the specific flavouring agent and the desired organoleptic effect. Typically, the amount of flavouring agent, if present in the composition, ranges from about 0.001% w/v to about 0.1 %.

Compositions

Water is the main solvent of the formulation. Typically, purified water for pharmaceutical use is used, which is available commercially, commonly obtained by distillation, ion exchange or any other suitable method from drinkable water. The exact percentage of water is not quantified, as it is adjusted to a certain final volume, to provide the desired concentration of every ingredient.

Preferably, the solvent of the suspension of the present invention consists of water, i.e., the composition preferably does not contain any additional organic solvent, in particular, it does not contain glycerine.

In an embodiment, the suspension of the present invention does not comprise a wetting agent selected from ethanol, isopropanol, propan-1-ol, glycerine, propylene glycol, 1,3-propanediol, and a combination thereof; even more particularly it does not comprise glycerine.

The composition of the present invention is in the form of a suspension. A suspension, as is well known in pharmaceutical formulation, is as a two-phase system consisting of an undissolved or immiscible solid dispersed in a liquid. Tadalafil is a poorly soluble drug which is present as a solid dispersed phase in the suspension.

The viscosity of the suspensions of the invention is generally comprised between 100 and 3000 cPs. Preferably, the viscosity of the suspensions of the invention is comprised between 125 and 2500 cPs, and more preferably is comprised between 150 and 1500 cPs.

In one embodiment, the present invention relates to a pharmaceutical composition for oral administration in the form of aqueous suspension which comprises:

a) tadalafil in an amount comprised between 0.01% and 5%, preferably comprised between 0.02% and 2.0%, more preferably comprised between 0.05% and 1.5%, still more preferably comprised between 0.08% and 1.2%, still more preferably comprised between 0.09% and 1.1% and still more preferably comprised between 0.1% and 1.0%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 1.50%, more preferably comprised between 0.20% and 1.00%, still more preferably comprised between 0.25% and 0.90%, still more preferably comprised between 0.30% and 0.85%, still more preferably comprised between 0.35% and 0.75%, still more preferably comprised between 0.40% and 0.65%, and still more preferably comprised between 0.45% and 0.60%;

e) water;

f) optionally a surfactant in an amount preferably comprised between 0.001% and about 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., g of each component per 100 ml of the suspension.

In one embodiment, the composition comprises the components a) to f) and components g) to j) are optional, corresponding to a preferred embodiment of the invention wherein the composition additionally comprises a surfactant.

In one embodiment, the composition comprises the components a) to g) and components h) to j) are optional, corresponding to a preferred embodiment of the invention wherein the composition additionally comprises a surfactant and a preservative.

In one embodiment, the composition comprises the components a) to h) and components i) to j) are optional, corresponding to a preferred embodiment of the invention wherein the composition additionally comprises a surfactant, a preservative and a pH adjusting agent.

In one particular embodiment, the composition consists of the above recited components a) to j), either compulsorily or optional, as above defined, i.e., it does not comprise any additional excipient or active ingredient.

In one embodiment, the present invention relates to a pharmaceutical composition for oral administration in the form of aqueous suspension which comprises:

a) tadalafil in an amount comprised between 0.01% and 0.40%, preferably comprised between 0.02% and 0.30%, more preferably comprised between 0.05% and 0.20%, still more preferably comprised between 0.08% and 0.15%, still more preferably comprised between 0.09% and 0.12% and still more preferably is about 0.1%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 0.75%, more preferably comprised between 0.25% and 0.65%, still more preferably comprised between 0.36% and 0.54%, still more preferably comprised between 0.40% and 0.50%, and still more preferably is about 0.45%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., grams of each component per 100 ml of the suspension.

In one embodiment, the composition comprises the components a) to f) and components g) to j) are optional, corresponding to a preferred embodiment of the invention wherein the composition additionally comprises a surfactant.

In one embodiment, the composition comprises the components a) to g) and components h) to j) are optional, corresponding to a preferred embodiment of the invention wherein the composition additionally comprises a surfactant and a preservative.

In one embodiment, the composition comprises the components a) to h) and components i) to j) are optional, corresponding to a preferred embodiment of the invention wherein the composition additionally comprises a surfactant, a preservative and a pH adjusting agent.

In one particular embodiment, the composition consists of the above recited components a) to j), either compulsorily or optional, as above defined, i.e., it does not comprise any additional excipient or active ingredient.

In one embodiment, the present invention relates to a pharmaceutical composition for oral administration in the form of aqueous suspension which comprises:

a) tadalafil in an amount comprised between 0.1% and 4.0%, preferably comprised between 0.2% and 3.0%, more preferably comprised between 0.5% and 2.0%, still more preferably comprised between 0.8% and 1.5%, still more preferably comprised between 0.9% and 1.2%, and still more preferably is about 1.0%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.20% and 1.50%, more preferably comprised between 0.30% and 0.95%, more preferably comprised between 0.42% and 0.80%, still more preferably comprised between 0.48% and 0.72%, still more preferably comprised between 0.55% and 0.65%, and still more preferably is about 0.60%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., grams of each component per 100 ml of the suspension.

In one embodiment, the composition comprises the components a) to f) and components g) to j) are optional, corresponding to a preferred embodiment of the invention wherein the composition additionally comprises a surfactant.

In one embodiment, the composition comprises the components a) to g) and components h) to j) are optional, corresponding to a preferred embodiment of the invention wherein the composition additionally comprises a surfactant and a preservative.

In one embodiment, the composition comprises the components a) to h) and components i) to j) are optional, corresponding to a preferred embodiment of the invention wherein the composition additionally comprises a surfactant, a preservative and a pH adjusting agent.

In one particular embodiment, the composition consists of the above recited components a) to j), either compulsorily or optional, as above defined, i.e., it does not comprise any additional excipient or active ingredient.

In one embodiment, the present invention relates to a pharmaceutical composition for oral administration in the form of aqueous suspension which comprises:

a) tadalafil in an amount comprised between 0.1% and 4.0%, preferably comprised between 0.2% and 3.0%, more preferably comprised between 0.5% and 2.0%, still more preferably comprised between 0.8% and 1.5%, still more preferably comprised between 0.9% and 1.2%, and still more preferably is about 1.0%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from α-cyclodextrin and β-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 0.75%, more preferably comprised between 0.20% and 0.60%, still more preferably comprised between 0.25% and 0.40%, and still more preferably is about 0.30%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., grams of each component per 100 ml of the suspension.

In one embodiment, the composition comprises the components a) to f) and components g) to j) are optional, corresponding to a preferred embodiment of the invention wherein the composition additionally comprises a surfactant.

In one embodiment, the composition comprises the components a) to g) and components h) to j) are optional, corresponding to a preferred embodiment of the invention wherein the composition additionally comprises a surfactant and a preservative.

In one embodiment, the composition comprises the components a) to h) and components i) to j) are optional, corresponding to a preferred embodiment of the invention wherein the composition additionally comprises a surfactant, a preservative and a pH adjusting agent.

In one particular embodiment, the composition consists of the above recited components a) to j), either compulsorily or optional, as above defined, i.e., it does not comprise any additional excipient or active ingredient.

Additionally, all the compositions disclosed above may also optionally comprise a colouring agent, in order to improve their appearance and make them more organoleptically appealing.

Any colouring agent suitable for use in pharmaceuticals may be used, as are well known in the art, for example, those disclosed under the section "Coloring Agents" of the bood "Handbook of Pharmaceutical Ingredients" *op. cit.*

Combination with tamsulosin or with dutasteride

The compositions of the invention, comprising tadalafil as the active ingredient in the form of an oral suspension, can optionally additionally comprise or be combined with one or more suitable additional active ingredients, for providing fixed-dose combination dosage forms.

One particularly useful therapeutic strategy is the combined use of tadalafil and tamsulosin for the treatment of patients with lower urinary tract symptoms (LUTS) related to benign prostatic hyperplasia (BPH), for example, as disclosed in Sebastianelli *et al. op. cit.* Another useful combination is tadalafil and dutasteride for the treatment of LUTS secondary to BPH.

Tamsulosin is the International Nonproprietary Name (INN) of the substance (R)-5-(2-((2-(2-ethoxyphenoxy) ethyl)amino)propyl)-2-methoxybenzenesulfonamide. Tamsulosin is an alfa1 adrenoreceptor antagonist and is used, also as monotherapy, for the treatment of lower urinary tract symptoms (LUTS) associated with benign prostatic hyperplasia (BPH). Tamsulosin may be used as the free base or as a pharmaceutically acceptable salt or solvate thereof. Tamsulosin is generally marketed as the hydrochloride salt.

Dutasteride is the International Nonproprietary Name (INN) of the substance (1S,3aS,3bS,5aR,9aR,9b-S,11aS)-N-[2,5-bis(trifluoromethyl)phenyl]-9a,11a-dimethyl-7-oxo-1,2,3,3a,3b,4,5,5a,6,9b,10,11-dodecahydroindeno [5,4-f]quinoline-1-carboxamide. Dutasteride is a $5\alpha$-reductase inhibitor which is generally used for the treatment of symptoms of benign prostatic hyperplasia (BPH). Dutasteride may be also used as a pharmaceutically acceptable salt or a solvate thereof. Preferably the free base is used.

Methods for preparing tamsulosin and dutasteride are well known in the art, for example, as disclosed in chapters 12 (Adrenoblockers) and 27 (Steroid Hormones), respectively, of the book R. Vardanyan and V. Hruby, Synthesis of best-seller drugs, Elsevier, 2016. Tamsulosin, and pharmaceutically acceptable salts thereof, specifically, tamsulosin hydrochloride, as well as dutasteride, are also commercially available from several suppliers.

The preparation of a fixed-dose dosage form comprising tadalafil and tamsulosin or comprising tadalafil and dutasteride is therapeutically advantageous, particularly, for increasing drug compliance. Furthermore, it is particularly useful include the advantages of the tadalafil suspension disclosed herein in the combined oral dosage form.

Therefore, another aspect of the present invention is a fixed-dose combination dosage form comprising the tadalafil aqueous suspension according to the present invention and tamsulosin, or a pharmaceutically acceptable salt thereof.

Tamsulosin is generally used in therapy in the form of modified-release oral dosage forms, since delayed or prolonged release has proved to be therapeutically advantageous for treating lower urinary tract symptoms (LUTS) associated with benign prostatic hyperplasia (BPH).

Therefore, the fixed-dose combination dosage form comprises preferably tamsulosin in the form of modified-release pellets.

Advantageously, for this aspect of the invention, the tadalafil suspension and the tamsulosin pellets in the combined dosage form are provided together, but physically separated, to be combined just before administration.

Surprisingly, the authors of the present invention have found that the tadalafil suspension according to the present invention has optimal properties, in particular, optimal viscosity and rheological properties, so it allows to be easily combined, if needed, with other solid ingredients in particulate form, in particular, with tamsulosin pellets, so a homogeneous mixture can be easily obtained with only gentle shaking, and which can be therefore be easily swallowed and has also good palatability.

Advantageously, this fixed-dose combination dosage form is adapted to deliver the following doses of both components:

- a dose of tadalafil comprised between 1 mg and 10 mg, preferably comprised between 2 mg and 8 mg, more preferably comprised between 3 mg and 7 mg, still more preferably comprised between 4 mg and 6 mg, and still more preferably of about 5 mg; and
- a dose of tamsulosin comprised between 0.05 mg and 1 mg, preferably comprised between 0.08 mg and 0.8 mg, more preferably comprised between 0.15 mg and 0.65 mg, still more preferably comprised between 0.30 mg and 0.50 mg, and still more preferably of about 0.4 mg.

A preferred fixed-dose combination dosage form comprises about 5 mg of tadalafil and about 0.4 mg of tamsulosin.

Advantageously, for example, this combination may be provided in mono-dose vials, wherein a suitable dose of tadalafil, as the tadalafil suspension of the invention, is placed in the vial, and a suitable dose of tamsulosin, preferably as tamsulosin pellets, is enclosed in the cap, which is provided with means to be perforated to release the pellets into the tadalafil suspension, before administration. Both components can be then easily mixed, with gentle shaking, to provide a homogeneous mixture of both ingredients.

Any concentration of the tadalafil suspension may be used for its combined use with tamsulosin. A suitable volume of the suspension is therefore calculated in order to provide the suitable dose, to be combined with tamsulosin.

Any type of modified-release tamsulosin pellets can be used to be combined with the tadalafil suspension.

As is well-known in the art, pharmaceutical pellets are multi-particulate drug delivery systems where the whole dose is divided into multiple subunits with spherical shape and narrow particle size distribution, within the range of about 0.1 mm to about 1.5 mm.

Typically, the pellets comprise a core, comprising the active ingredient, i.e., tamsulosin, and one or more coating layers, comprising one or more modifying release polymers. Said modified-release film coatings, may be further categorized as either delayed-release (e.g., gastro-resistant) or extended-release coatings.

As is well known in the art, film-coating processes involve the application of a coating liquid onto the core containing the active ingredient. Film-coating formulations typically comprise polymer, plasticizer, and solvent/vehicle. Among some common modified-release coating polymers are some cellulose derivatives such as ethylcellulose and cellulose acetate; methylmethacrylate copolymers; methacrylic acid copolymers; and phthalate esters, such as hydroxypropyl methylcellulose phthalate, cellulose acetate phthalate and, poly(vinyl acetate phthalate; among many others.

The core may be prepared, for example, using inert spheres, typically, sugar spheres, and coating a suitable tadalafil solution or suspension onto them, for example, an aqueous solution or suspension containing tadalafil, together with a binder and, optionally other excipients. Alternatively, the core of the pellets may be prepared by extrusion/spheronization process, as is well-known in the art.

Additionally, uncoated modified-release pellets may be also used, which may be prepared by embedding tamsulosin within a modified-release matrix composition and producing the pellets by extrusion/spheronization.

Some examples of tamsulosin delayed-release pellets which can be used in the present invention are, for example, those disclosed in the in the following patent documents: EP-A-3473245, WO-A-2004/043449, WO-A-2005/060939, WO-A-2005/004851, WO-A-2007/021101, WO-A-2007/117110, WO-A-2014/203137, WO-A-2016/155682 and WO-A-2018/030862.

In one embodiment, one aspect of the invention relates to a fixed-dose combination dosage form comprising:

A) A pharmaceutical composition for oral administration in the form of aqueous suspension which comprises:

a) tadalafil in an amount comprised between 0.01 % w/v and 5 %, preferably comprised between 0.02% and 2.0%, more preferably comprised between 0.05% and 1.5%, still more preferably comprised between 0.08% and 1.2%, still more preferably comprised between 0.09% and 1.1% and still more preferably comprised between 0.1% and 1.0%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 1.50%, more preferably comprised between 0.20% and 1.00%, still more preferably comprised between 0.25% and 0.90%, still more preferably comprised between 0.30% and 0.85%, still more preferably comprised between 0.35% and 0.75%, still more preferably comprised between 0.40% and 0.65%, and still more preferably comprised between 0.45% and 0.60%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., g of each component per 100 ml of the suspension;

wherein, preferably, the composition comprises the components a) to f) and components g) to j) are optional, more preferably the composition comprises the components a) to g) and components h) to j) are optional, and still more preferably the composition comprises the components a) to h) and components i) to j) are optional;

and wherein, preferably, the composition consists of the above recited components a) to j), either compulsorily or optionally, as above defined, i.e., it does not comprise any additional excipient or active ingredient;

and

B) tamsulosin, or a pharmaceutically acceptable salt thereof, wherein preferably tamsulosin is in the form of modified-release pellets.

In another embodiment, one aspect of the invention relates to a combined oral dosage form comprising:

A) A pharmaceutical composition for oral administration in the form of aqueous suspension which comprises:

a) tadalafil in an amount comprised between 0.01% and 0.40%, preferably comprised between 0.02% and 0.30%, more preferably comprised between 0.05% and 0.20%, still more preferably comprised between 0.08% and 0.15%, still more preferably comprised between 0.09% and 0.12% and still more preferably is about 0.1%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 0.75%, more preferably comprised between 0.25% and 0.65%, still more preferably comprised between 0.36% and 0.54%, still more preferably comprised between 0.40% and 0.50%, and still more preferably is about 0.45%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., g of each component per 100 ml of the suspension;

wherein, preferably, the composition comprises the components a) to f) and components g) to j) are optional, more preferably the composition comprises the components a) to g) and components h) to j) are optional, and still more preferably the composition comprises the components a) to h) and components i) to j) are optional;

and wherein, preferably, the composition consists of the above recited components a) to j), either compulsorily or optionally, as above defined, i.e., it does not comprise any additional excipient or active ingredient;

and

B) tamsulosin, or a pharmaceutically acceptable salt thereof, wherein preferably tamsulosin is in the form of modified-release pellets.

Another aspect of the present invention is a fixed-dose combination dosage form comprising the tadalafil aqueous suspension according to the present invention and dutasteride, or a pharmaceutically acceptable salt thereof.

Preferably, dutasteride is used as the free base.

Dutasteride is easily degraded when it is in contact with tadalafil. Therefore, advantageously, tadalafil and dutasteride in the combined dosage form are provided together, but physically separated to avoid dutasteride degradation. For example, both active ingredients may be placed in separate compartments within the same package, to be combined only shortly before the ingestion. Alternatively, dutasteride may be included within the tadalafil suspension, but using some kind of protective barrier to avoid interaction, for example, using coated dutasteride particles or including dutasteride within oil globules, as a microemulsion formulation, for example.

As discussed above, the tadalafil suspension according to the present invention has optimal properties, in particular, optimal viscosity and rheological properties, so it allows to be easily combined with other solid ingredients in particulate form, for example, with dutasteride particles, provided as powder or granules, for example. In this way, a homogeneous mixture comprising tadalafil and dutasteride can be easily obtained with only gentle shaking, and which can be therefore be easily swallowed and has also good palatability.

Advantageously, this fixed-dose combination dosage form is adapted to deliver the following doses of both components:

- a dose of tadalafil comprised between 1 mg and 10 mg, preferably comprised between 2 mg and 8 mg, more preferably comprised between 3 mg and 7 mg, still more preferably comprised between 4 mg and 6 mg, and still more preferably of about 5 mg; and

- a dose of dutasteride comprised between 0.05 mg and 1.5 mg, preferably comprised between 0.1 mg and 1.0 mg, more preferably comprised between 0.2 mg and 0.8 mg, still more preferably comprised between 0.4 mg and 0.6 mg, and still more preferably of about 0.5 mg.

A preferred fixed-dose combination dosage form comprises about 5 mg of tadalafil and about 0.5 mg of dutasteride.

Advantageously, for example, this combination may be provided in mono-dose vials, wherein a suitable dose of tadalafil, as the tadalafil suspension of the invention, is placed in the vial, and a suitable dose of dutasteride, as powder or granulate, for example, is enclosed in the cap, which is provided with means to be perforated to release the dutasteride powder or granulate into the tadalafil suspension, before administration. Both components can be then easily mixed, with gentle shaking, to provide a homogeneous mixture of both ingredients.

Any concentration of the tadalafil suspension may be used for its combined use with dutasteride. A suitable volume of the suspension is therefore calculated in order to provide the suitable dose, to be combined with dutasteride.

Preferably, dutasteride is in the form powder or granules, to be combined with tadalafil suspension. Any particular formulation of said powder or granules may be used. Typically, said powder or granules provide immediate-release of dutasteride.

As the amount of dutasteride to be administered in each combined dose is low, typically ranging from about 0.05 to about 1.5 mg, the formulation of dutasteride powder or granules preferably includes at least one diluent, which acts as a bulking agent, in order to have an optimal amount of powder or granules to be homogeneously mixed with the tadalafil suspension, said amount is typically comprised between 50 mg and 1000 mg. Suitable diluents are, for example, dextrose, lactose, sucrose, maltose, mannitol, maltitol, sorbitol, xylitol, calcium phosphate, calcium carbonate, sodium carbonate, calcium sulfate, microcrystalline cellulose, powdered cellulose, starch or pregelatinized starch, among many others, or mixtures thereof.

Typically, for preparing dutasteride powder formulation, dutasteride in powder form is mixed with excipients, typically with a diluent. Optionally, further excipients can be added, such as glidants or colouring agents, for example, as is well known in the art. Suitable glidants include, among others, colloidal silicon dioxide, magnesium oxide, magnesium silicate, sodium stearate and talc.

When dutasteride is in form or granules, the formulation preferably also comprises a binder, for example, selected from gelatin, povidone, hypromellose, hydroxypropyl cellulose, polyethylene glycol, sucrose, starch and pregelatinized starch, among many others, and mixtures thereof.

The granules can be prepared following standard methods, either by dry granulation or wet granulation. Suitable methods for preparing powder and granule formulations are disclosed, for example, in Chapter 28 ("Powders, granules and granulation") of the book of M.E. Aulton and K.M.G. Taylor, *op. cit.*

In one embodiment, this aspect of the invention relates to a fixed-dose combination dosage form comprising:

A) A pharmaceutical composition for oral administration in the form of aqueous suspension which comprises:

a) tadalafil in an amount comprised between 0.01 % w/v and 5 %, preferably comprised between 0.02% and 2.0%, more preferably comprised between 0.05% and 1.5%, still more preferably comprised between 0.08% and 1.2%, still more preferably comprised between 0.09% and 1.1% and still more preferably comprised between 0.1% and 1.0%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 1.50%, more preferably comprised between 0.20% and 1.00%, still more preferably comprised between 0.25% and 0.90%, still more preferably comprised between 0.30% and 0.85%, still more preferably comprised between 0.35% and 0.75%, still more preferably comprised between 0.40% and 0.65%, and still more preferably comprised between 0.45% and 0.60%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., g of each component per 100 ml of the suspension;

wherein, preferably, the composition comprises the components a) to f) and components g) to j) are optional, more preferably the composition comprises the components a) to g) and components h) to j) are optional, and still more preferably the composition comprises the components a) to h) and components i) to j) are optional;

and wherein, preferably, the composition consists of the above recited components a) to j), either compulsorily or optionally, as above defined, i.e., it does not comprise any additional excipient or active ingredient;

and

B) dutasteride, or a pharmaceutically acceptable salt thereof, wherein preferably dutasteride is in the form of powder or granules.

In another embodiment, one aspect of the invention relates to a combined oral dosage form comprising:

A) A pharmaceutical composition for oral administration in the form of aqueous suspension which comprises:

a) tadalafil in an amount comprised between 0.01% and 0.40%, preferably comprised between 0.02% and 0.30%, more preferably comprised between 0.05% and 0.20%, still more preferably comprised between 0.08% and 0.15%, still more preferably comprised between 0.09% and 0.12% and still more preferably is about 0.1%;

b) cyclodextrin, in an amount preferably comprised between 0.01% and 0.25%, more preferably comprised between 0.02% and 0.20%, and still more preferably comprised between 0.03% and 0.10%; wherein the cyclodextrin is preferably selected from $\alpha$-cyclodextrin and $\beta$-cyclodextrin;

c) coprocessed microcrystalline cellulose and sodium carboxymethyl cellulose (MCC-NaCMC), in an amount preferably comprised between 0.5% and 2.0%, more preferably comprised between 0.6% and 1.4%, still more preferably comprised between 0.7% and 1.3%, still more preferably comprised between 0.8% and 1.2%, still more preferably comprised between 0.9% and 1.1%, and still more preferably is about 1.0%;

d) xanthan gum in an amount preferably comprised between 0.10% and 0.75%, more preferably comprised between 0.25% and 0.65%, still more preferably comprised between 0.36% and 0.54%, still more preferably comprised between 0.40% and 0.50%, and still more preferably is about 0.45%;

e) water;

f) optionally a surfactant in an amount preferably comprised between 0.001% and 2%, more preferably comprised between 0.010% and 0.100%, still more preferably comprised between 0.020% and 0.085%, and still more preferably comprised between 0.030% and 0.090%, wherein the surfactant is preferably selected from sodium lauryl sulfate and docusate sodium, and more preferably is sodium lauryl sulfate;

g) optionally a preservative in an amount preferably comprised between 0.001% and 5%, more preferably comprised between 0.01% and 1.00%, still more preferably comprised between 0.05% and 0.50%, wherein the preservative is preferably selected from butyl paraben, sodium butyl paraben, ethyl paraben, sodium ethyl paraben, methyl paraben, sodium methyl paraben, propyl paraben, sodium propyl paraben, benzoic acid, sodium benzoate, potassium benzoate, benzalkonium chloride, benzethonium chloride, benzyl alcohol, bronopol, chlorhexidine, chlorocresol, chloroxylenol, imidurea, cresol, phenol, sorbic acid, potassium sorbate, thimerosal and mixtures thereof, more preferably is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate and mixtures thereof, still more preferably is selected from sodium methyl paraben, sodium propyl paraben and mixtures thereof, and still more preferably is a mixture of sodium methyl paraben and sodium propyl paraben;

h) optionally a pH adjusting agent to adjust the pH of the composition to a value comprised between 2.5 and 7, preferably comprised between 4.0 and 6.5, more preferably comprised between 5.5 and 6; wherein the pH adjusting agent is preferably citrate buffer;

i) optionally a sweetening agent in an amount preferably comprised between 0.0001% and 0.5%, wherein the sweetening agent is preferably selected from aspartame, acesulfame potassium, alitame, neohesperidin dihydrochalcone, neotame, saccharin, saccharin sodium, sucralose, thaumatin, and mixtures thereof; more preferably is selected from aspartame, acesulfame potassium, saccharin and saccharin sodium; and

j) optionally, a flavouring agent, preferably in an amount comprised between 0.001% and 0.1%;

wherein the percentages are expressed as weight/volume (w/v), i.e., g of each component per 100 ml of the suspension;

wherein, preferably, the composition comprises the components a) to f) and components g) to j) are optional, more preferably the composition comprises the components a) to g) and components h) to j) are optional, and still more preferably the composition comprises the components a) to h) and components i) to j) are optional;

and wherein, preferably the composition consists of the above recited components a) to j), either compulsorily or optionally, as above defined, i.e., it does not comprise any additional excipient or active ingredient;

and

B) dutasteride, or a pharmaceutically acceptable salt thereof, wherein preferably dutasteride is in the form of powder or granules.

Preparation process

Another aspect of the invention is a process for preparing the composition.

A suitable process for preparing the composition of the invention comprises the following steps:

(i) mixing cyclodextrin, coprocessed MCC-NaCMC, and xanthan gum with one part of total water to obtain a homogeneous mixture;

(ii) separately mixing tadalafil with another part of total water, to obtain a homogeneous mixture;

(iii) adding the mixture of step (ii) to the mixture of step (i);

(iv) adding the rest of water.

All ingredients are added under continuous stirring. For example, steps (i) and (ii) may be performed in stainless steel reactors provided with agitation system.

In step (i) the exact proportion of water used to prepare the first mixture is not critical, and may be, for example, from about 30% to about 75% of total water.

When the composition comprises a pH adjusting agent, a preservative and/or a sweetening agent, they are preferably also added in step (i).

Typically, after adding coprocessed MCC-NaCMC, it is activated, typically by several stirring steps, according the manufacturer's instructions. For example, by subjecting the mixture to two consecutive stirring steps, first at a moderate stirring speed, for example, of maximum 800 r.p.m. for about 10-60 minutes, and then to a second more vigorous stirring step, for example, at about 1000-1500 r.p.m. for about 2-15 minutes. Typically, between both stirring steps and afterwards, the mixture is left to stand unstirred for about 5-60 minutes.

In step (ii) the amount of water used to prepare the second mixture is also not critical, and may be, for example, from about 5% to about 25% of total water.

The mixture obtained in step (ii) is a suspension.

When the composition comprises a surfactant, it is typically added in step (ii), together with tadalafil.

If there is a flavouring agent, it preferably is added after combining mixtures (i) and (ii).

The final suspension obtained is then dispensed into suitable containers.

For example, the suspension can be packaged in high-density polyethylene bottles, provided with a suitable dosing system such as, for example, a dose pump adapted to dispense a fixed volume of the suspension with each press, or any other suitable measuring device, for example, a graduated cup, oral syringe or dropper.

In one embodiment, the dosing device is a dosing pump adapted to dispense a fixed volume of the suspension with each press.

In another embodiment, the dosing device is a graduated syringe.

Preferably, the dosing device, combined with a suitable concentration of tadalafil in the suspension, is adapted to deliver doses of tadalafil comprised between 1 mg and 50 mg of tadalafil, preferably comprised between 2,5 and 20 mg of tadalafil, for example, doses of about 2.5 mg, 5 mg, 10 mg or 20 mg.

Use of the composition

The tadalafil composition according to the present invention can be suitably used for any one of the known tadalafil therapeutic indications.

Surprisingly, as shown in Example 5, the suspension according to the present invention, characterized by the specific combination of suspension agents, was able to provide a solubility profile which is analogous to that of the commercial Cialis® product, despite being formulated in a different form, namely, as film-coated tablets. Therefore, the present invention is capable of providing the same therapeutic effects as Cialis®, but in a more convenient and advantageous dosage form.

Its presentation in the form of an aqueous suspension facilitates the oral administration, while also allowing the dose to be adjusted to the particular requirements of each therapeutic indication and/or of each specific patient.

Therefore, another aspect of the invention is the pharmaceutical composition of the invention for use in therapy.

In one particular embodiment, the pharmaceutical composition according to the present invention is indicated for the treatment of masculine erectile dysfunction.

Erectile dysfunction, also known as masculine sexual dysfunction, is understood to mean the persistent inability of a male to achieve or maintain an erection of the penis sufficient to have satisfactory sexual activity.

A particular aspect of the invention is, therefore, the pharmaceutical composition of the invention for use in the treatment of masculine erectile dysfunction, or a method of treatment of masculine erectile dysfunction comprising administering to a patient in need thereof the pharmaceutical composition of the invention.

Typically, doses of about 10 mg or about 20 mg of tadalafil are used, taken prior to anticipated sexual activity. Alternatively, once daily regimen of about 2.5 mg or about 5 mg may be also used. The skilled in the art will have no difficulty in selecting the suitable therapeutically effective dose and/or dosage regimen for this therapeutic indication.

In another embodiment, the pharmaceutical composition according to the present invention is indicated for the treatment of benign prostatic hyperplasia (BPH).

As is well known, benign prostatic hyperplasia (BPH) is an age-related phenomenon associated with prostatic enlargement and bladder outlet obstruction. The treatment of BPH typically relates to treatment of signs and symptoms of BPH, in order to improve symptoms and reduce the risk of acute urinary retention and risk for BPH-related surgery.

Another aspect of the invention is, therefore, the pharmaceutical composition of the invention for use in the treatment of benign prostatic hyperplasia, or a method of treatment of benign prostatic hyperplasia comprising administering to a patient in need thereof the pharmaceutical composition of the invention.

Typically, doses of about 5 mg of tadalafil are used in the treatment of BPH. The skilled in the art will have no difficulty in selecting the suitable therapeutically effective dose and/or dosage regimen for this therapeutic indication.

Another aspect of the invention is the oral dosage form comprising tadalafil and tamsulosin for use in therapy.

A more particular aspect is the oral dosage form comprising tadalafil and tamsulosin for use in the treatment of benign prostatic hyperplasia, or a method of treatment of benign prostatic hyperplasia, comprising administering to a patient in need thereof the oral dosage form comprising tadalafil and tamsulosin.

Another aspect of the invention is the oral dosage form comprising tadalafil and dutasteride for use in therapy.

A more particular aspect is the oral dosage form comprising tadalafil and dutasteride for use in the treatment of benign prostatic hyperplasia, or a method of treatment of benign prostatic hyperplasia, comprising administering to a patient in need thereof the oral dosage form comprising tadalafil and dutasteride.

The invention relates to the following embodiments:

1.- Pharmaceutical composition for oral administration in the form of aqueous suspension which comprises:

    a) tadalafil in an amount comprised between 0.01 % w/v and 5 % w/v;
    b) cyclodextrin;
    c) coprocessed microcrystalline cellulose and sodium carboxymethyl cellulose (MCC-NaCMC);
    d) xanthan gum; and
    e) water.

2.- Composition according to embodiment 1, characterized in that the amount of tadalafil is comprised 0.02% w/v and 2.0% w/v, preferably comprised between 0.05% w/v and 1.5% w/v, more preferably comprised between 0.08% w/v and 1.2% w/v, still more preferably comprised between 0.09% w/v and 1.1% w/v and still more preferably comprised between 0.1% w/v and 1.0% w/v.

3.- Composition according to embodiment 1, characterized in that the amount of tadalafil is comprised between 0.01% w/v and 0.40% w/v, preferably comprised between 0.02% w/v and 0.30% w/v, more preferably comprised between 0.05% w/v and 0.20% w/v, still more preferably comprised between 0.08% w/v and 0.15% w/v, still more preferably comprised between 0.09% w/v and 0.12% w/v and still more preferably is about 0.1% w/v.

4.- Composition according to embodiment 1, characterized in that the amount of tadalafil is comprised between 0.1% w/v and 4.0% w/v, preferably comprised between 0.2% w/v and 3.0% w/v, more preferably comprised between 0.5% w/v and 2.0% w/v, still more preferably comprised between 0.8% w/v and 1.5% w/v, still more preferably comprised between 0.9% w/v and 1.2% w/v, and still more preferably is about 1.0% w/v.

5.- Composition according to any one of embodiments 1 to 4, characterized in that the amount of cyclodextrin is comprised between 0.01% w/v and 0.25% w/v, preferably comprised between 0.02% w/v and 0.20% w/v, and more preferably comprised between 0.03% w/v and 0.10% w/v.

6.- Composition according to any one of embodiments 1 to 5, characterized in that the cyclodextrin is selected from $\alpha$-cyclodextrin, $\beta$-cyclodextrin, $\gamma$-cyclodextrin and highly-branched cyclodextrin (HBCD).

7.- Composition according to embodiment 6, characterized in that the cyclodextrin is selected from $\alpha$-cyclodextrin and $\beta$-cyclodextrin, and preferably is $\alpha$-cyclodextrin.

8.- Composition according to any one of embodiments 1 to 7, characterized in that the amount of coprocessed CMC-NaCMC is comprised between 0.5% w/v and 2.0% w/v, preferably comprised between 0.6% w/v and 1.4% w/v, more preferably comprised between 0.7% w/v and 1.3% w/v, still more preferably comprised between 0.8% w/v and 1.2% w/v, still more preferably comprised between 0.9% w/v and 1.1% w/v, and still more preferably is about 1.0% w/v.

9.- Composition according to any one of embodiments 1 to 8, characterized in that the amount of xanthan gum is comprised between 0.10% w/v and 1.50% w/v, preferably comprised between 0.20% w/v and 1.00% w/v, more preferably comprised between 0.25% w/v and 0.90% w/v, still more preferably comprised between 0.30% w/v and 0.85% w/v, still more preferably comprised between 0.35% w/v and 0.75% w/v, still more preferably comprised between 0.40% w/v and 0.65% w/v, and still more preferably comprised between 0.45% w/v and 0.60% w/v.

10.- Composition according to embodiment 9, characterized in that the amount of xanthan gum is comprised between 0.10% w/v and 0.75% w/v, preferably comprised between 0.20% w/v and 0.60% w/v, more preferably comprised between 0.25% w/v and 0.40% w/v, and still more preferably is about 0.30% w/v.

11.- Composition according to embodiment 9, characterized in that the amount of xanthan gum is comprised between 0.10% w/v and 0.75% w/v, preferably comprised between 0.25% w/v and 0.65% w/v, more preferably comprised between 0.36% w/v and 0.54% w/v, still more preferably comprised between 0.40% w/v and 0.50% w/v, and still more preferably is about 0.45% w/v.

12.- Composition according to embodiment 9, characterized in that the amount of xanthan gum is comprised between 0.20% w/v and 1.50% w/v, preferably comprised between 0.30% w/v and 0.95% w/v, more preferably comprised between 0.42% w/v and 0.80% w/v, still more preferably comprised between 0.48% w/v and 0.72% w/v, still more preferably comprised between 0.55% w/v and 0.65% w/v, and still more preferably is about 0.60% w/v.

13.- Composition according to any one of embodiments 1 to 12, characterized in that it comprises a surfactant, preferably in an amount comprised between 0.001% and 2%.

14.- Composition according to embodiment 13, characterized in that the surfactant is an anionic surfactant, preferably selected from sodium lauryl sulfate and docusate sodium.

15.- Composition according to embodiment 14, characterized in that the surfactant is sodium lauryl sulfate, preferably in an amount comprised between 0.010% w/v and 0.100% w/v, preferably comprised between 0.020% w/v and 0.085% w/v, and more preferably comprised between 0.030% w/v and 0.090% w/v.

16.- Composition according to any one of embodiments 1 to 15, characterized in that the composition comprises a preservative, preferably in a concentration comprised between 0.001% w/v and 5% w/v, more preferably comprised between 0.01% w/v and 1.00% w/v, and still more preferably comprised between 0.05% w/v and 0.50% w/v.

17.- Composition according to embodiment 16, characterized in that the preservative is selected from butyl paraben, butyl paraben sodium, propyl paraben, propyl paraben sodium, methyl paraben, methyl paraben sodium, benzoic acid, sodium benzoate, potassium benzoate, sorbic acid, potassium sorbate, and mixtures thereof, preferably selected from sodium methyl paraben, sodium propyl paraben and mixtures thereof, and more preferably is a mixture of sodium methyl paraben and sodium propyl paraben.

18.- Composition according to any one of embodiments 1 to 17, characterized in that it comprises a pH adjusting agent to adjust the pH of the composition to a value comprised between 2.5 and 7, preferably comprised between 4.0 and 6.5, more preferably comprised between 5.5 and 6.

19.- Composition according to embodiment 18, characterized in that the pH adjusting agent is a buffering agent, which is citrate buffer, acetate buffer, citrate-phosphate buffer, Tris buffer, and phosphate buffer.

20.- Composition according to embodiment 19, characterized in that the pH adjusting agent is citrate buffer.

21.- Composition according to any one of embodiments 1 to 20, characterized in that it comprises a sweetening agent, preferably selected from aspartame, acesulfame potassium, alitame, neohesperidin dihydrochalcone, neotame, saccharin, saccharin sodium, sucralose, thaumatin, or mixtures thereof, more preferably selected from aspartame, acesulfame potassium, saccharin and saccharin sodium.

22.- Composition according to embodiment 21, characterized in that the amount of the sweetening agent is comprised between 0.0001% w/v and 0.5% w/v.

23.- Composition according to any one of embodiments 1 to 22, characterized in that it comprises a flavouring agent.

24.- Composition according to embodiment 23, characterized in that the amount of the flavouring agent is comprised between 0.001% w/v and 0.1% w/v.

25.- Composition according to any one of embodiments 1 to 24, characterized in that the composition consists of: a) tadalafil; b) cyclodextrin; c) coprocessed MCC-NaCMC; d) xanthan gum; e) water; f) optionally, a surfactant; g) optionally, a preservative; h) optionally, a pH adjusting agent; i) optionally, a sweetening agent; and j) optionally, a flavouring agent.

26.- Composition according to embodiment 25, characterized in that the composition consists of: a) tadalafil; b) cyclodextrin; c) coprocessed MCC-NaCMC; d) xanthan gum; e) water; f) a surfactant, g) optionally, a preservative; h) optionally, a pH adjusting agent; i) optionally, a sweetening agent; and j) optionally, a flavouring agent.

27.- Composition according to embodiment 26, characterized in that the composition consists of: a) tadalafil; b) cyclodextrin; c) coprocessed MCC-NaCMC; d) xanthan gum; e) water; f) a surfactant; g) a preservative; h) optionally, a pH adjusting agent; i) optionally, a sweetening agent; and j) optionally, a flavouring agent.

28.- Composition according to embodiment 27, characterized in that the composition consists of: a) tadalafil; b) cyclodextrin; c) MCC-NaCMC; d) xanthan gum; e) water; f) a surfactant; g) a preservative; h) a pH adjusting agent; i) optionally, a sweetening agent; and j) optionally, a flavouring agent.

29.- Composition according to any one of embodiments 1 to 28, characterized in that the composition additionally contains: k) optionally, a colouring agent.

30.- A composition according to any one of embodiments 1 to 29, characterized in that the composition does not contain a wetting agent selected from ethanol, isopropanol, propan-1-ol, glycerine, propylene glycol, 1,3-propanediol, and a combination thereof; more particularly it does not comprise glycerine.

31.- Fixed-dose combination dosage form comprising tadalafil aqueous suspension according to any one of embodiments 1 to 30 and a second drug selected from tamsulosin, or a pharmaceutically acceptable salt thereof, and dutasteride, or a pharmaceutically acceptable salt thereof.

32.- Fixed-dose combination dosage form according to embodiment 31, characterized in that it comprises tamsulosin, or a pharmaceutically acceptable salt thereof, preferably comprises tamsulosin in the form of tamsulosin hydrochloride.

33.- Fixed-dose combination dosage form according to embodiment 32, characterized in that tamsulosin is in the form of modified-release pellets.

34.- Fixed-dose combination dosage form according to any one of embodiments 32 or 33, characterized in that it is adapted to deliver the following doses both components:

- a dose of tadalafil comprised between 1 mg and 10 mg, preferably comprised between 2 mg and 8 mg, more preferably comprised between 3 mg and 7 mg, still more preferably comprised between 4 mg and 6 mg, and still more preferably of about 5 mg; and
- a dose of tamsulosin comprised between 0.05 mg and 1 mg, preferably comprised between 0.08 mg and 0.8 mg, more preferably comprised between 0.15 mg and 0.65 mg, still more preferably comprised between 0.30 mg and 0.50 mg, and still more preferably of about 0.4 mg.

35.- Fixed-dose combination dosage form according to any one of embodiments 32 to 34, characterized in that it is provided in mono-dose vials, wherein tadalafil suspension is placed in the vial and tamsulosin is enclosed in the cap.

36.- Fixed-dose combination dosage form according to embodiment 31, characterized in that it comprises dutasteride, or a pharmaceutically acceptable salt thereof, preferably comprises dutasteride free base.

37.- Fixed-dose combination dosage form according to embodiment 36, characterized in that dutasteride is in the form of powder or granules.

38.- Fixed-dose combination dosage form according to any one of embodiments 36 or 37, characterized in that it is adapted to deliver the following doses both components:

- a dose of tadalafil comprised between 1 mg and 10 mg, preferably comprised between 2 mg and 8 mg, more preferably comprised between 3 mg and 7 mg, still more preferably comprised between 4 mg and 6 mg, and still more preferably of about 5 mg; and
- a dose of dutasteride comprised between 0.05 mg and 1.5 mg, preferably comprised between 0.1 mg and 1.0 mg, more preferably comprised between 0.2 mg and 0.8 mg, still more preferably comprised between 0.4 mg and 0.6 mg, and still more preferably of about 0.5 mg.

39.- Fixed-dose combination dosage form according to any one of embodiments 36 to 38, characterized in that it is provided in mono-dose vials, wherein tadalafil suspension is placed in the vial and dutasteride is enclosed in the cap.

40.- Process for preparing the composition according to any one of embodiments 1 to 30, characterized in that it comprises the following steps:

(i) mixing cyclodextrin, coprocessed MCC-NaCMC, and xanthan gum with one part of total water to obtain a homogeneous mixture;
(ii) separately mixing tadalafil with another part of total water, to obtain a homogeneous mixture;
(iii) adding the mixture of step (ii) to the mixture of step (i);
(iv) adding the rest of water.

41.- Composition according to any one of embodiments 1 to 30 or fixed-dose combination dosage form according to any one of embodiments 31 to 39 for use in therapy.

42.- Composition according to any one of embodiments 1 to 30 for use in the treatment of masculine erectile dysfunction or benign prostatic hyperplasia.

43.- Fixed-dose combination dosage form according to any one of embodiments 31 to 39 for use in the treatment of benign prostatic hyperplasia.

**Examples**

Example 1: Suspension compositions comprising 1% tadalafil

Examples 1A to 1D having a strength of 1% tadalafil, were prepared using the ingredients listed in the following table:

| Ingredient | % (w/v) (g/100 ml) | | | | |
|---|---|---|---|---|---|
| | 1A | 1B | 1C | 1D | 1E |
| Tadalafil | 1.000 | 1.000 | 1.000 | 1.000 | 1.000 |

(continued)

| Ingredient | % (w/v) (g/100 ml) | | | | |
|---|---|---|---|---|---|
| | 1A | 1B | 1C | 1D | 1E |
| Cyclodextrin | 0.100 | 0.100 | 0.120 | 0.100 | 0.033 |
| MCC-NaCMC | 1.000 | 1.000 | 1.000 | 1.200 | 1.000 |
| Xanthan Gum | 0.500 | 0.600 | 0.600 | 0.600 | 0.300 |
| Sodium lauryl sulfate (SLS) | 0.03-0.09 | 0.03-0.09 | 0.03-0.09 | 0.03-0.09 | 0.03-0.09 |
| Citrate buffer | 2-3 | 2-3 | 2-3 | 2-3 | 2-3 |
| Sweetener | 0.0001-0.5 | 0.0001-0.5 | 0.0001-0.5 | 0.0001-0.5 | 0.0001-0.5 |
| Preservative | 0.05-0.5 | 0.05-0.5 | 0.05-0.5 | 0.05-0.5 | 0.05-0.5 |
| Purified water | q.s.100ml | q.s.100ml | q.s.100ml | q.s.100ml | q.s.100ml |

The MCC-NaCMC used was VivaPur MCG 811P (JRS Pharma). Tadalafil had a particle size of Dv90 11.2 microns (compositions 1A-1D) or 10.5 microns (composition 1E). For compositions 1A, 1B, 1C and 1D, the cyclodextrin used was a HBCD (Cluster Dextrin, Glico Nutrition), and for composition 1E, the cyclodextrin was an $\alpha$-cyclodextrin (Cavamax® W6 Pharma, Wacker Chemie AG).

For preparing the compositions 1A, 1B, 1C, 1D and 1E a similar process was used, as disclosed below.

A stainless-steel reactor equipped with agitation system was filled with purified water equivalent to about 50% of the final batch, and then anhydrous citric acid, sodium citrate, sodium methyl paraben, sodium propyl paraben, aspartame and cyclodextrin were stepwise added to the reactor under continuous agitation until total dissolution. Subsequently, MCC-NaCMC and xanthan gum were slowly added and the mixture was stirred suitably in order to activate MCC-NaCMC.

A secondary stainless-steel reactor equipped with agitation was filled with about 15% of total water and then SLS and tadalafil were stepwise added under agitation and the mixture was homogenised under stirring for about 10 minutes. This mixture was mixed with the contents of the first reactor, carefully rinsing the reactor with water to collect all the substances adhered to the walls, and finally adding the remaining water to the final volume, maintaining continuous stirring, until obtaining a homogeneous suspension.

The final product, in all four cases, was a white or almost white viscous suspension (230-2550 cPs). The pH value was about 5.7.

The suspensions were filled into PET amber bottles of 30 ml, equipped either with a pump suitable for dispensing 0.5 ml of the suspension with each stroke of the pump, or with a plug closed with a child-resistant cap with a syringe suitable to measure the required dose.

Example 2: Suspension compositions comprising 0.1% tadalafil

Examples 2A, 2B and 2C, having a strength of 0.1% tadalafil, were prepared using the ingredients listed in the following table:

| Ingredient | % (w/v) (g/100ml) | | | |
|---|---|---|---|---|
| | 2A | 2B | 2C | 2D |
| Tadalafil | 0.100 | 0.100 | 0.100 | 0.100 |
| Cyclodextrin | 0.100 | 0.100 | 0.100 | 0.100 |
| MCC-NaCMC | 1.000 | 1.000 | 1.000 | 1.000 |
| Xanthan Gum | 0.450 | 0.360 | 0.540 | 0.450 |
| Sodium lauryl sulfate (SLS) | 0.03-0.09 | 0.03-0.09 | 0.03-0.09 | 0.03-0.09 |
| Citrate buffer | 2-3 | 2-3 | 2-3 | 2-3 |
| Sweetener | 0.0001-0.5 | 0.0001-0.5 | 0.0001-0.5 | 0.0001-0.5 |
| Preservative | 0.05-0.5 | 0.05-0.5 | 0.05-0.5 | 0.05-0.5 |
| Purified water | q.s.100ml | q.s.100ml | q.s.100ml | q.s.100ml |

The MCC-NaCMC used in all compositions was VivaPur MCG 811P (JRS Pharma). Tadalafil had a particle size of Dv90 11.2 microns.

The cyclodextrin used in compositions 2A, 2B and 2C was a HBCD (Cluster Dextrin, Glico Nutrition), and the cyclodextrin used in composition 2D was an α-cyclodextrin (Cavamax® W6 Pharma, Wacker Chemie AG).

For preparing the compositions 2A, 2B, 2C, and 2D, the process followed was analogous to that described in Example 1.

The final product, in all three cases, was a white or almost white viscous suspension (270-420 cPs). The pH value was about 5.7 for all products (2A, 2B, 2C and 2D).

Example 3: Fixed dose combination comprising a 0.1% suspension of tadalafil and delayed-release tamsulosin pellets.

Fixed-dose combination of tadalafil and tamsulosin was prepared in mono-dose vials, using the suspension composition of Example 2 and modified-release tamsulosin pellets.

The tamsulosin modified-release pellets were prepared analogously as disclosed in WO-A-2018/030862.

The content of tamsulosin in the final delayed-release pellets was of 0.125 wt%.

Extemporary mono-dose vials were filled with 5 ml of the suspension of Example 2A, equivalent to 5 mg of tadalafil. The caps were filled with 320 mg of the tamsulosin pellets, equivalent to 0.4 mg of tamsulosin.

The caps of those extemporary mono-dose vials were adapted to be pierced, immediately before the administration, to allow the pellets to be released into the vial. The mixture was thus prepared to be homogenised by gently shaken and be ready for administration.

Example 4: Fixed dose combination comprising a 0.1% suspension of tadalafil and dutasteride.

Fixed-dose combination of tadalafil and dutasteride was prepared in mono-dose vials, using the suspension composition of Example 2 and dutasteride powder formulation.

The dutasteride powder formulation was prepared by thoroughly mixing dutasteride (0.125 wt%), lactose anhydrous (between about 59 and 75 wt%), microcrystalline cellulose (between about 24 and 40 wt% and colloidal silicon dioxide (between about 0.5 and 1.0 wt%).

Extemporary mono-dose vials were filled with 5 ml of the suspension of Example 2A, equivalent to 5 mg of tadalafil. The caps were filled with 400 mg of the dutasteride powder formulation, equivalent to 0.5 mg of dutasteride.

The caps of those extemporary mono-dose vials were adapted to be pierced, immediately before the administration, to allow the dutasteride powder to be released into the vial. The mixture was thus prepared to be homogenised by gently shaken and be ready for administration.

Example 5: Dissolution assay of the suspensions of the invention compared to Cialis® film-coated tablets

For assessing the dissolution behaviour of tadalafil in the suspensions prepared, compared to Cialis® commercial film-coated tablets, comparative dissolution assays were performed.

For Examples 1A, 1B, 1C and 1D, the comparative dissolution assays were performed by placing 2 ml of each suspension or one 20 mg Cialis® film-coated tablet, for comparison, in a vessel.

For Example 1E, the comparative dissolution assay was performed by placing 0.5 ml of the suspension or one 5 mg Cialis® film-coated tablet, for comparison, in a vessel.

For Examples 2A, 2B, 2C, and 2D, the comparative dissolution assays were performed by placing 5 ml of each suspension or one 5 mg Cialis® film-coated tablet, for comparison, in a vessel. The comparative assay for Example 2D was performed independently from those for Examples 2A, 2B and 2C and, therefore, an independent dissolution assay of the reference compound Cialis® was performed for this comparative test.

For each assay, 900 ml of dissolution medium was used.

The dissolution media were prepared using purified water and adjusting the pH to the following values 1.2, 4.5 and 6.8, using HCl (pH 1.2) or acetate buffer (pH 4.5) or phosphate buffer (pH 6.8), for example, as disclosed in in the European Pharmacopoeia 6.0 (Eur. Phar. 6.0), Chapter 2.9.3: *Dissolution Test for Solid Dosage Forms,* plus 0.5% SLS in each one.

The dissolution assays were performed with stirring at 50 rpm. Samples were taken at the following time points: 0, 5, 10 15, 20, 30 and 45 minutes and the amount of tadalafil dissolved in the samples was analysed using HPLC, using the equipment and conditions as disclosed below:

| | |
|---|---|
| Column | Luna C$_{18}$. dimensions 250 x 4.6 mm, 5 μm |

(continued)

| Temperature | 25 °C |
| Mobile phase flow | 1.0 ml/minute |
| Detection | UV absorption, $\lambda$ Tadalafil = 285 nm |
| Injection volume | 20 $\mu$l |
| Duration of the chromatogram | 7 minutes |
| Mobile phase | (A/B) 50:50 |
| | A: H2O + 0.2% TFA |
| | B: Acetonitrile |

The assay was repeated 6 times for each sample and the average value was calculated. For Cialis® film-coated tablets, the dissolution assay was repeated 12 times and the average value was also calculated.

The percentage of tadalafil dissolved at each time point, at the three pH conditions is summarized in the following tables:

| | Example 1A | | | Example 1B | | | Example 1C | | |
|---|---|---|---|---|---|---|---|---|---|
| t (min) | 1.2 | 4.5 | 6.8 | 1.2 | 4.5 | 6.8 | 1.2 | 4.5 | 6.8 |
| 0 | 0.00 | 0.00 | 0.00 | 0.00 | 0.00 | 0.00 | 0.00 | 0.00 | 0.00 |
| 5 | 61.6 | 68.5 | 62.6 | 61.8 | 69.2 | 66.2 | 69.4 | 65.9 | 73.5 |
| 10 | 68.7 | 80.4 | 68.2 | 68.7 | 75.0 | 74.0 | 77.0 | 73.9 | 78.9 |
| 15 | 72.0 | 82.1 | 75.4 | 70.7 | 80.2 | 80.8 | 80.1 | 78.9 | 82.4 |
| 20 | 73.0 | 85.6 | 80.5 | 73.3 | 84.1 | 87.1 | 82.3 | 82.8 | 85.5 |
| 30 | 77.6 | 90.9 | 85.0 | 74.4 | 86.5 | 89.6 | 83.4 | 87.7 | 88.6 |
| 45 | 80.0 | 97.2 | 91.1 | 76.4 | 89.9 | 92.3 | 85.7 | 90.6 | 90.2 |

| | Example 1D | | | Reference (Cialis® 20 mg) | | |
|---|---|---|---|---|---|---|
| t (min) | 1.2 | 4.5 | 6.8 | 1.2 | 4.5 | 6.8 |
| 0 | 0.00 | 0.00 | 0.00 | 0.00 | 0.00 | 0.00 |
| 5 | 60.7 | 74.8 | 65.8 | 50.8 | 51.2 | 47.3 |
| 10 | 68.8 | 81.8 | 72.6 | 67.3 | 73.6 | 71.8 |
| 15 | 72.9 | 85.6 | 76.7 | 75.9 | 83.3 | 81.5 |
| 20 | 75.6 | 88.0 | 83.0 | 82.9 | 87.7 | 86.6 |
| 30 | 81.5 | 92.9 | 86.2 | 88.2 | 91.4 | 91.9 |
| 45 | 81.2 | 97.3 | 91.7 | 90.1 | 94.7 | 95.1 |

| | Example 1E | | | Reference (Cialis® 5 mg) | | |
|---|---|---|---|---|---|---|
| t (min) | 1.2 | 4.5 | 6.8 | 1.2 | 4.5 | 6.8 |
| 0 | 0.00 | 0.00 | 0.00 | 0.00 | 0.00 | 0.00 |
| 5 | 62.7 | 71.6 | 66.8 | 52.8 | 53.2 | 55.7 |
| 10 | 76.1 | 83.7 | 78.8 | 70.8 | 74.3 | 75.7 |
| 15 | 81.7 | 89.3 | 86.6 | 79.8 | 85.0 | 87.7 |
| 20 | 85.1 | 92.3 | 90.5 | 85.0 | 90.0 | 92.8 |
| 30 | 87.4 | 95.9 | 94.6 | 90.1 | 94.8 | 97.5 |
| 45 | 92.2 | 98.4 | 97.3 | 92.5 | 96.6 | 100.6 |

|  | Example 2A | | | Example 2B | | |
|---|---|---|---|---|---|---|
| t (min) | 1.2 | 4.5 | 6.8 | 1.2 | 4.5 | 6.8 |
| 0 | 0.00 | 0.00 | 0.00 | 0.00 | 0.00 | 0.00 |
| 5 | 68.3 | 78.0 | 76.0 | 68.5 | 76.3 | 76.4 |
| 10 | 71.0 | 80.3 | 78.5 | 71.0 | 80.1 | 78.6 |
| 15 | 72.8 | 83.3 | 80.9 | 71.9 | 82.3 | 81.1 |
| 20 | 74.7 | 86.1 | 83.2 | 74.0 | 84.2 | 83.0 |
| 30 | 75.1 | 88.2 | 86.0 | 75.3 | 86.2 | 85.9 |
| 45 | 74.7 | 91.3 | 89.6 | 78.1 | 89.0 | 88.0 |

|  | Example 2C | | | Reference (Cialis® 5 mg) | | |
|---|---|---|---|---|---|---|
| t (min) | 1.2 | 4.5 | 6.8 | 1.2 | 4.5 | 6.8 |
| 0 | 0.00 | 0.00 | 0.00 | 0.00 | 0.00 | 0.00 |
| 5 | 63.2 | 68.7 | 66.7 | 45.0 | 62.9 | 60.2 |
| 10 | 66.9 | 74.6 | 73.3 | 62.1 | 74.5 | 76.2 |
| 15 | 68.8 | 77.7 | 76.2 | 73.0 | 81.2 | 84.9 |
| 20 | 69.9 | 80.3 | 77.9 | 77.8 | 85.0 | 86.6 |
| 30 | 70.7 | 83.2 | 80.7 | 82.3 | 87.9 | 90.5 |
| 45 | 73.7 | 85.3 | 83.5 | 83.2 | 90.6 | 92.4 |

|  | Example 2D | | | Reference (Cialis® 5 mg) | | |
|---|---|---|---|---|---|---|
| t (min) | 1.2 | 4.5 | 6.8 | 1.2 | 4.5 | 6.8 |
| 0 | 0.00 | 0.00 | 0.00 | 0.00 | 0.00 | 0.00 |
| 5 | 72.7 | 82.8 | 79.0 | 52.8 | 53.2 | 55.7 |
| 10 | 78.4 | 85.5 | 83.4 | 70.8 | 74.3 | 75.7 |
| 15 | 77.7 | 90.2 | 89.7 | 79.8 | 85.0 | 87.7 |
| 20 | 77.7 | 89.9 | 90.3 | 85.0 | 90.0 | 92.8 |
| 30 | 80.5 | 97.9 | 94.5 | 90.1 | 94.8 | 97.5 |
| 45 | 80.2 | 99.2 | 99.7 | 92.5 | 96.6 | 100.6 |

For quantitatively comparing the dissolution similarity between the reference compound (Cialis® 20 mg film-coated tablet or Cialis® 5 mg film-coated tablet) and the suspensions according to the present invention, the similarity factor $f_2$, was used, as disclosed in Moore et al., Mathematical comparison of curves with an emphasis on in vitro dissolution profiles, Pharm. Tech., 1996, 20(6), 64-74 or in Helmy et al., In vitro dissolution similarity as a surrogate for in vivo bioavailability and therapeutic equivalence, Dissolution Technologies, August 2016, 32-39, and in the Guideline on the investigation of bioequivalence CMPM/EWP/QWP/1401/98 Rev. 1.

As is well known in the art, the factor $f_2$ is a measure of the similarity between the dissolution curves obtained from the test and reference products. This factor is calculated from the mean of the dissolution profiles at each of the sampling times using the following equation:

$$f_2 = 50 \times \log\left\{\left[1 + \left(\frac{1}{n}\right)\sum_{t=1}^{n}(R_t - P_t)\right]^{-0.5} \times 100\right\}$$

where:

- n = number of sampling times
- Rt = average percentage dissolved at time t of the reference drug
- Pt = average percentage dissolved at time t of the test drug

Those points where the percentage of dissolved drug exceeds 85% are not taken into account for $f_2$ calculation.

The comparison was performed for each sample vs. the corresponding reference compound (either Cialis® 5mg or 20 mg) and at each pH value (1.2, 4.5 and 6.8)

The value of $f_2$ varies from 0 to 100. If the value is equal to or greater than 50, the product is considered to meet the similarity factor in relation to the reference product.

Furthermore, when in the dissolution assay more than 85% of the substance is already dissolved at 15 minutes in both formulations, there is no need to calculate the exact value of $f_2$ and it is considered that the similarity factor is also met.

The following table summarizes the value of the similarity factor $f_2$ for the assayed compositions. The values "n.c." mean that the similarity factor $f_2$ is not calculated because more than 85% dissolution is already reached at 15 minutes.

|  | pH=1.2 | pH=4.5 | pH=6.8 |
|---|---|---|---|
| Example 1A | 53.87 | 51.31 | 52.26 |
| Example 1B | 51.44 | 51.37 | 50.96 |
| Example 1C | 50.30 | 54.51 | 43.32 |
| Example 1D | 59.26 | 41.92 | 50.53 |
| Example 1E | 61.84 | n.c. (>85% diss.) | n.c. (>85% diss.) |
| Example 2A | 47.43 | 54.25 | 53.65 |
| Example 2B | 47.91 | 56.67 | 53.15 |
| Example 2C | 48.85 | 68.57 | 57.26 |
| Example 2D | 47.26 | n.c. (>85% diss.) | n.c. (>85% diss.) |

It can be observed that for all the suspensions assayed, the similarity factor is met for at least two of the three pH conditions assayed, either because the value of $f_2$ is more than 50 or because more than 85% dissolution is reached at 15 minutes.

Example 6: Stability assays

Compositions of Example 1 (1A, 1B, 1C, 1D and 1E) and Example 2 (2A, 2B, 2C and 2D) were tested for stability and, to this end, they were stored up to 9 months at 25°C $\pm$ 2°C and 60% $\pm$ 5% relative humidity (RH) in some cases, and, up to 6 months at 40°C $\pm$ 2°C and 75% $\pm$ 5% RH in all of them. Samples of each composition were analysed after 1, 3, 6 and 9 months by HPLC with UV detection for tadalafil, tadalafil related substances and other impurities. Other stability parameters were also assessed in the assay, as appearance, viscosity, pH, microbiological testing, preservative content and dissolution of tadalafil.

It was found that all tested samples fulfilled the required stability criteria.

**Claims**

1. Pharmaceutical composition for oral administration in the form of aqueous suspension which comprises:

    a) tadalafil in an amount comprised between 0.01 % w/v and 5 % w/v;
    b) cyclodextrin;
    c) coprocessed microcrystalline cellulose and sodium carboxymethyl cellulose (MCC-NaCMC);
    d) xanthan gum; and
    e) water.

2. Composition according to claim 1, **characterized in that** the amount of tadalafil is comprised between 0.02% w/v and 2.0% w/v, preferably comprised between 0.05% w/v and 1.5% w/v, more preferably comprised between 0.08% w/v and 1.2% w/v, still more preferably comprised between 0.09% w/v and 1.1% w/v and still more preferably comprised between 0.1% w/v and 1.0% w/v.

3. Composition according to claims 1 to 2, **characterized in that** the amount of cyclodextrin is comprised between 0.01% w/v and 0.25% w/v, preferably comprised between 0.02% w/v and 0.20% w/v, and more preferably comprised between 0.03% w/v and 0.10% w/v.

4. Composition according to claim 3, **characterized in that** the cyclodextrin is selected from a $\alpha$-cyclodextrin, $\beta$-cyclodextrin, $\gamma$-cyclodextrin, highly-branched cyclodextrin (HBCD), and mixtures thereof, preferably is selected from a $\alpha$-cyclodextrin and a $\beta$-cyclodextrin.

5. Composition according to any one of claims 1 to 4, **characterized in that** the amount of coprocessed CMC-NaCMC is comprised between 0.5% w/v and 2.0% w/v, preferably comprised between 0.6% w/v and 1.4% w/v, more preferably comprised between 0.7% w/v and 1.3% w/v, still more preferably comprised between 0.8% w/v and 1.2% w/v, still more preferably comprised between 0.9% w/v and 1.1% w/v, and still more preferably is about 1.0% w/v.

6. Composition according to any one of claims 1 to 5, **characterized in that** the amount of xanthan gum is comprised between 0.10% w/v and 1.50% w/v, preferably comprised between 0.20% w/v and 1.00% w/v, more preferably comprised between 0.25% w/v and 0.90% w/v, still more preferably comprised between 0.30% w/v and 0.85% w/v, still more preferably comprised between 0.35% w/v and 0.75% w/v, still more preferably comprised between 0.40% w/v and 0.65% w/v, and still more preferably comprised between 0.45% w/v and 0.60% w/v.

7. Composition according to any one of claims 1 to 6, **characterized in that** it comprises a surfactant, preferably sodium lauryl sulfate in an amount comprised between 0.010% w/v and 0.100% w/v, more preferably comprised between 0.020% w/v and 0.085% w/v, and still more preferably comprised between 0.030% w/v and 0.090% w/v.

8. Composition according to any one of claims 1 to 7, **characterized in that** the composition comprises a preservative, preferably in a concentration comprised between 0.001% w/v and 5% w/v.

9. Composition according to any one of claims 1 to 8, **characterized in that** it comprises a pH adjusting agent to adjust the pH of the composition to a value comprised between 2.5 and 7, preferably comprised between 4.0 and 6.5, more preferably comprised between 5.5 and 6.

10. Composition according to claim 9, **characterized in that** the pH adjusting agent is a buffering agent, which is selected from citrate buffer, acetate buffer, citrate-phosphate buffer, Tris buffer, and phosphate buffer.

11. Fixed-dose combination dosage form comprising tadalafil aqueous suspension according to any one of claims 1 to 10 and a second drug selected from tamsulosin, or a pharmaceutically acceptable salt thereof, and dutasteride, or a pharmaceutically acceptable salt thereof.

12. Fixed-dose combination dosage form according to claim 11, **characterized in that** it comprises tamsulosin, preferably as tamsulosin hydrochloride, which is preferably in the form of modified-release pellets.

13. Process for preparing the composition according to any one of claims 1 to 10, **characterized in that** it comprises the following steps:

> (i) mixing cyclodextrin, coprocessed MCC-NaCMC, and xanthan gum with one part of total water to obtain a homogeneous mixture;
> (ii) separately mixing tadalafil with another part of total water, to obtain a homogeneous mixture;
> (iii) adding the mixture of step (ii) to the mixture of step (i);
> (iv) adding the rest of water.

14. Composition according to any one of claims 1 to 10 or fixed-dose combination dosage form according to any one of claims 11 to 12 for use in therapy.

15. Composition according to any one of claims 1 to 10 for use in the treatment of masculine erectile dysfunction or in the treatment of benign prostatic hyperplasia or fixed-dose combination dosage form according to any one of claims 11 to 12 for use in the treatment of benign prostatic hyperplasia.

**Patentansprüche**

1. Pharmazeutische Zusammensetzung zur oralen Verabreichung in der Form einer wässrigen Suspension, die umfasst:

   a) Tadalafil in einer Menge, die zwischen 0,01 % w/v und 5 % w/v liegt;
   b) Cyclodextrin;
   c) koprozessierte mikrokristalline Cellulose und Natriumcarboxymethylcellulose (MCC-NaCMC);
   d) Xanthangummi; und
   e) Wasser.

2. Zusammensetzung nach Anspruch 1, **dadurch gekennzeichnet, dass** die Menge an Tadalafil zwischen 0,02% w/v und 2,0% w/v liegt, vorzugsweise zwischen 0,05% w/v und 1,5% w/v liegt, weiter bevorzugt zwischen 0,08% w/v und 1,2% w/v liegt, noch weiter bevorzugt zwischen 0,09% w/v und 1,1% w/v liegt und noch weiter bevorzugt zwischen 0,1% w/v und 1,0% w/v liegt.

3. Zusammensetzung nach den Ansprüchen 1 bis 2, **dadurch gekennzeichnet, dass** die Menge an Cyclodextrin zwischen 0,01% w/v und 0,25% w/v liegt, vorzugsweise zwischen 0,02% w/v und 0,20% w/v liegt und weiter bevorzugt zwischen 0,03% w/v und 0,10% w/v liegt.

4. Zusammensetzung nach Anspruch 3, **dadurch gekennzeichnet, dass** das Cyclodextrin ausgewählt ist aus einem $\alpha$-Cyclodextrin, $\beta$-Cyclodextrin, $\gamma$-Cyclodextrin, hochverzweigten Cyclodextrin (HBCD) und Gemischen davon, vorzugsweise ausgewählt ist aus einem $\alpha$-Cyclodextrin und einem $\beta$-Cyclodextrin.

5. Zusammensetzung nach einem der Ansprüche 1 bis 4, **dadurch gekennzeichnet, dass** die Menge an koprozessiertem CMC-NaCMC zwischen 0,5% w/v und 2,0% w/v liegt, vorzugsweise zwischen 0,6% w/v und 1,4% w/v liegt, weiter bevorzugt zwischen 0,7% w/v und 1,3% w/v liegt, noch weiter bevorzugt zwischen 0,8% w/v und 1,2% w/v liegt, noch weiter bevorzugt zwischen 0,9% w/v und 1,1% w/v liegt und noch weiter bevorzugt etwa 1,0% w/v beträgt.

6. Zusammensetzung nach einem der Ansprüche 1 bis 5, **dadurch gekennzeichnet, dass** die Menge an Xanthangummi zwischen 0,10% w/v und 1,50% w/v liegt, vorzugsweise zwischen 0,20% w/v und 1,00% w/v liegt, weiter bevorzugt zwischen 0,25% w/v und 0,90% w/v liegt, noch weiter bevorzugt zwischen 0,30% w/v und 0,85% w/v liegt, noch weiter bevorzugt zwischen 0,35% w/v und 0,75% w/v liegt, noch weiter bevorzugt zwischen 0,40% w/v und 0,65% w/v liegt und noch weiter bevorzugt zwischen 0,45% w/v und 0,60% w/v liegt.

7. Zusammensetzung nach einem der Ansprüche 1 bis 6, **dadurch gekennzeichnet, dass** sie ein Tensid, vorzugsweise Natriumlaurylsulfat, in einer Menge umfasst, die zwischen 0,010% w/v und 0,100% w/v liegt, weiter bevorzugt zwischen 0,020% w/v und 0,085% w/v liegt und noch weiter bevorzugt zwischen 0,030% w/v und 0,090% w/v liegt.

8. Zusammensetzung nach einem der Ansprüche 1 bis 7, **dadurch gekennzeichnet, dass** die Zusammensetzung ein Konservierungsmittel umfasst, vorzugsweise in einer Konzentration, die zwischen 0,001% und 5% w/v liegt.

9. Zusammensetzung nach einem der Ansprüche 1 bis 8, **dadurch gekennzeichnet, dass** sie ein pH-Einstellmittel umfasst, um den pH-Wert der Zusammensetzung auf einen Wert einzustellen, der zwischen 2,5 und 7 liegt, vorzugsweise zwischen 4,0 und 6,5 liegt, weiter bevorzugt zwischen 5,5 und 6 liegt.

10. Zusammensetzung nach Anspruch 9, **dadurch gekennzeichnet, dass** das pH-Einstellmittel ein Puffermittel ist, das aus Citratpuffer, Acetatpuffer, Citrat-Phosphat-Puffer, Tris-Puffer und Phosphatpuffer ausgewählt ist.

11. Festdosierte Kombinationsdosierungsform, umfassend eine wässrige Tadalafil-Suspension nach einem der Ansprüche 1 bis 10 und ein zweites Arzneimittel, das aus Tamsulosin oder einem pharmazeutisch verträglichen Salz davon und Dutasterid oder einem pharmazeutisch verträglichen Salz davon ausgewählt ist.

12. Festdosierte Kombinationsdosierungsform nach Anspruch 11, **dadurch gekennzeichnet, dass** sie Tamsulosin, vorzugsweise als Tamsulosinhydrochlorid, umfasst, das vorzugsweise in Form von Pellets mit modifizierter Freisetzung vorliegt.

13. Verfahren zur Herstellung der Zusammensetzung nach einem der Ansprüche 1 bis 10, **dadurch gekennzeichnet,**

**dass** es die folgenden Schritte umfasst:

(i) Mischen von Cyclodextrin, koprozessiertem MCC-NaCMC und Xanthangummi mit einem Teil der Gesamtwassermenge, um ein homogenes Gemisch zu erhalten;
(ii) separates Mischen von Tadalafil mit einem weiteren Teil der Gesamtwassermenge, um ein homogenes Gemisch zu erhalten;
(iii) Hinzugeben des Gemischs aus Schritt (ii) zu dem Gemisch aus Schritt (i);
(iv) Hinzugeben der restlichen Wassermenge.

14. Zusammensetzung nach einem der Ansprüche 1 bis 10 oder festdosierte Kombinationsdosierungsform nach einem der Ansprüche 11 bis 12 zur Verwendung in einer Therapie.

15. Zusammensetzung nach einem der Ansprüche 1 bis 10 zur Verwendung bei der Behandlung von männlicher erektiler Dysfunktion oder bei der Behandlung von benigner Prostatahyperplasie oder festdosierte Kombinationsdosierungsform nach einem der Ansprüche 11 bis 12 zur Verwendung bei der Behandlung von benigner Prostatahyperplasie.

**Revendications**

1. Composition pharmaceutique pour administration orale sous la forme d'une suspension aqueuse qui comprend :

a) du tadalafil en une quantité comprise entre 0,01 % p/v et 5 % p/v,
b) de la cyclodextrine,
c) de la cellulose microcristalline et de la carboxyméthyl cellulose sodique co-traitées (MCC-NaCMC),
d) de la gomme de xanthane, et
e) de l'eau.

2. Composition selon la revendication 1, **caractérisée en ce que** la quantité de tadalafil est comprise entre 0,02 % p/v et 2,0 % p/v, de manière préférée comprise entre 0,05 % p/v et 1,5 % p/v, de manière plus préférée comprise entre 0,08 % p/v et 1,2 % p/v, de manière encore plus préférée comprise entre 0,09 % p/v et 1,1 % p/v et de manière encore plus préférée comprise entre 0,1 % p/v et 1,0 % p/v.

3. Composition selon les revendications 1 à 2, **caractérisée en ce que** la quantité de cyclodextrine est comprise entre 0,01 % p/v et 0,25 % p/v, de manière préférée comprise entre 0,02 % p/v et 0,20 % p/v, et de manière plus préférée comprise entre 0,03 % p/v et 0,10 % p/v.

4. Composition selon la revendication 3, **caractérisée en ce que** la cyclodextrine est choisie parmi une $\alpha$-cyclodextrine, une $\beta$-cyclodextrine, une $\gamma$-cyclodextrine, une cyclodextrine hautement ramifiée (HBCD) et des mélanges de celles-ci, et est de préférence choisie parmi une $\alpha$-cyclodextrine et une $\beta$-cyclodextrine.

5. Composition selon l'une quelconque des revendications 1 à 4, **caractérisée en ce que** la quantité de CMC-NaCMC co-traitée est comprise entre 0,5 % p/v et 2,0 % p/v, de manière préférée comprise entre 0,6 % p/v et 1,4 % p/v, de manière plus préférée comprise entre 0,7 % p/v et 1,3 % p/v, de manière encore plus préférée comprise entre 0,8 % p/v et 1,2 % p/v, de manière encore plus préférée comprise entre 0,9 % p/v et 1,1 % p/v, et est de manière encore plus préférée d'environ 1,0 % p/v.

6. Composition selon l'une quelconque des revendications 1 à 5, **caractérisée en ce que** la quantité de gomme de xanthane est comprise entre 0,10 % p/v et 1,50 % p/v, de manière préférée comprise entre 0,20 % p/v et 1,00 % p/v, de manière plus préférée comprise entre 0,25 % p/v et 0,90 % p/v, de manière encore plus préférée comprise entre 0,30 % p/v et 0,85 % p/v, de manière encore plus préférée comprise entre 0,35 % p/v et 0,75 % p/v, de manière encore plus préférée comprise entre 0,40 % p/v et 0,65 % p/v, et de manière encore plus préférée comprise entre 0,45 % p/v et 0,60 % p/v.

7. Composition selon l'une quelconque des revendications 1 à 6, **caractérisée en ce qu'**elle comprend un tensioactif, de préférence du laurylsulfate de sodium, en une quantité comprise entre 0,010 % p/v et 0,100 % p/v, de manière plus préférée comprise entre 0,020 % p/v et 0,085 % p/v, et de manière encore plus préférée comprise entre 0,030 % p/v et 0,090 % p/v.

8. Composition selon l'une quelconque des revendications 1 à 7, **caractérisée en ce que** la composition comprend un conservateur, de préférence à une concentration comprise entre 0,001 % p/v et 5 % p/v.

9. Composition selon l'une quelconque des revendications 1 à 8, **caractérisée en ce qu'**elle comprend un agent d'ajustement de pH pour ajuster le pH de la composition à une valeur comprise entre 2,5 et 7, de manière préférée comprise entre 4,0 et 6,5, de manière plus préférée comprise entre 5,5 et 6.

10. Composition selon la revendication 9, **caractérisée en ce que** l'agent d'ajustement du pH est un agent tampon, qui est choisi parmi un tampon citrate, un tampon acétate, un tampon citrate-phosphate, un tampon Tris et un tampon phosphate.

11. Forme galénique de combinaison à dose fixe comprenant une suspension aqueuse de tadalafil selon l'une quelconque des revendications 1 à 10 et un second médicament choisi parmi la tamsulosine, ou un sel de celle-ci acceptable du point de vue pharmaceutique, et du dutastéride, ou un sel de celui-ci acceptable du point de vue pharmaceutique.

12. Forme galénique de combinaison à dose fixe selon la revendication 11, **caractérisée en ce qu'**elle comprend de la tamsulosine, de préférence sous forme de chlorhydrate de tamsulosine, qui se présente de préférence sous la forme de pastilles à libération modifiée.

13. Procédé pour préparer la composition selon l'une quelconque des revendications 1 à 10, **caractérisé en ce qu'**il comprend les étapes suivantes :

   i) mélanger de la cyclodextrine, de la MCC-NaCMC co-traitée et de la gomme de xanthane avec une partie de l'eau totale pour obtenir un mélange homogène,
   ii) mélanger séparément du tadalafil avec une autre partie de l'eau totale pour obtenir un mélange homogène,
   iii) ajouter le mélange de l'étape ii) au mélange de l'étape i),
   iv) ajouter le reste d'eau.

14. Composition selon l'une quelconque des revendications 1 à 10 ou forme galénique de combinaison à dose fixe selon l'une quelconque des revendications 11 à 12, pour une utilisation en thérapie.

15. Composition selon l'une quelconque des revendications 1 à 10, pour une utilisation dans le traitement d'une dysfonction érectile masculine ou dans le traitement d'une hyperplasie prostatique bénigne, ou forme galénique de combinaison à dose fixe selon l'une quelconque des revendications 11 à 12, pour une utilisation dans le traitement d'une hyperplasie prostatique bénigne.

## REFERENCES CITED IN THE DESCRIPTION

**Patent documents cited in the description**

- WO 2020039263 A
- WO 2018142189 A
- EP 3473245 A
- WO 2004043449 A
- WO 2005060939 A
- WO 2005004851 A
- WO 2007021101 A
- WO 2007117110 A
- WO 2014203137 A
- WO 2016155682 A
- WO 2018030862 A

**Non-patent literature cited in the description**

- **SEBASTIANELLI et al.** *J. Clin. Med.*, 2019, vol. 8, 1126
- **PRABHU et al.** *Drugs Ther. Perspect.*, 2019, vol. 35, 181-184
- **P.J. SHESKEY** ; **W.G. COOK** ; **C.G. CABLE**. Handbook of Pharmaceutical Excipients. Pharmaceutical Press, 2017
- **J.P REMINGTON** ; **A. R. GENARO**. Remington, The Science and Practice of Pharmacy. Lippincott, Williams & Wilkins, 2000
- **M.E. AULTON** ; **K.M.G. TAYLOR**. Aulton's Pharmaceutics, the design and manufacture of medicines. Churchill Livingstone Elsevier, 2013
- *CHEMICAL ABSTRACTS*, 171596-29-5
- Drugs for Treatment of Erectile disfunction. **R. VARDANYAN** ; **V. HRUBY**. Synthesis of best-seller drugs. Elsevier, 2016
- **TANAKA et al.** *Carbohydr. Res.*, 1996, vol. 295, 91-101
- **TAKATA et al.** *J. Bacteriol.*, 1996, vol. 178, 1600-1606
- **TAKATA et al.** *J. Ferment. Bioeng.*, 1997, vol. 84, 119-123
- **FUJI et al.** *Biocatal. Biotransformation*, 2003, vol. 21, 167-172
- **MOORE et al.** Mathematical comparison of curves with an emphasis on in vitro dissolution profiles. *Pharm. Tech.*, 1996, vol. 20 (6), 64-74
- **HELMY et al.** In vitro dissolution similarity as a surrogate for in vivo bioavailability and therapeutic equivalence. *Dissolution Technologies*, August 2016, 32-39